# EUROPEAN PATENT SPECIFICATION

(11) **EP 3 685 804 B1**
(45) Date of publication and mention of the grant of the patent: **09.12.2026**
(21) Application number: 20163356.7
(22) Date of filing: 25.03.2016
(51) Int. Cl.: A61F 2/28, A61F 2/30, A61F 2/40

(54) **JOINT PROSTHESIS SPACER**
GELENKPROTHESENABSTANDHALTER
ESPACEUR DE PROTHÈSE D'ARTICULATION

(43) Date of publication of application: 29.07.2020
(62) Document number(s) of the earlier application(s) in accordance with Art. 76 EPC: 16305351.5 / 3 222 253
(73) Proprietor: TORNIER, 38330 Montbonnot-Saint-Martin (FR)
(72) Inventor: NEICHEL, Nicolas, 38330 Montbonnot Saint Martin (FR); VENNIN, Matthieu, 38330 Montbonnot Saint Martin (FR); LEON, Cédric, 38330 Montbonnot Saint Martin (FR); WALCH, Gilles, 38330 Montbonnot Saint Martin (FR); DERANSART, Pierric, 38330 Montbonnot Saint Martin (FR); BOILEAU, Pascal, 06300 Nice (FR)
(74) Representative: Lavoix

(56) References cited:
- EP-A1- 2 335 655
- US-A1- 2013 110 470
- US-A1- 2014 257 499
- US-A1- 2015 202 045

## Description

The present invention relates to a joint prosthesis spacer. The invention also relates to systems comprising such a joint prosthesis spacer.

### BACKGROUND OF THE INVENTION

### Field of the Invention

This application is directed to patient specific methods and devices useful in shoulder arthroplasty and other joint and orthopedic surgery.

### Description of the Related Art

Arthroplasty is the standard of care for the treatment of advanced shoulder joint problems, such as severe arthritis. Shoulder arthroplasty can replicate the anatomical form of a joint, with a spherical component mounted on the proximal humerus and a concave surface mounted on the glenoid region of the scapula. Certain patients benefit from a reverse shoulder reconstruction in which a spherical component is mounted to the scapula and a concave surface is positioned on the proximal humerus. Articulation of the spherical component on the concave surface provides the patient with improved arm motion.

One leading reverse shoulder technique, known as bony increased offset reverse shoulder arthroplasty or BIO-RSA provides improved outcomes for patients. BIO-RSA involves placing a spacer between the glenoid region of the scapula and a spherical joint component that is coupled with the glenoid. Among other benefits, BIO-RSA can improve range of motion, limit notching of the scapula, and correct bone deficiency.

Surgeons currently use standard spacers in BIO-RSA, for example with medial and lateral surfaces parallel to each other, disposed at a 12.5 degree angle relative to each other, and with limited options for thickness, such as 7mm or 10mm. In cases where the glenoid region surface is worn and may have missing bone portions a spacer with a flat surface will not seat properly against the glenoid and early loosening of the implant can result from improper seating. Further, in cases where the glenoid region surface is worn and may have missing bone portions excessive reaming of the bone may be needed to establish a flat surface on which a spacer can be seated. However excessive reaming of the glenoid surface can remove the dense subchondral bone and expose the soft and porous cancellous bone which is a poor seating surface for the spacer. Early loosening of the implant can be a consequence of excessive reaming. What is needed is a spacer than can conform to the worn glenoid surface without excessive reaming of the scapula.

US 2014/257499 discloses an implant assembly comprising a bone augment and an articulating member. The bone augment includes a bone-engaging surface and a coupling surface. The articulating member is configured to couple with the bone augment. The bone-engaging surface can have any suitable size and shape to fill a bone defect at an implant location for the bone augment, such as either a patient's glenoid or humerus. The bone-engaging surface can be a patient-specific surface sized and shaped to fill and be complimentary to a bone defect of a specific patient. A 3D model may be created based on imaging data, and a mold is formed based on the 3D model. A surgeon may then use the mold to form the bone augment. The mold may be a two-piece mold that allows a surgeon to insert bone graft or any suitable biocompatible material into the mold and then apply pressure to the mold to form the bone augment.

EP 2 335 655 discloses a prosthesis assembly for use with a scapula, including a glenoid bearing support and a bearing. The glenoid bearing support includes a glenoid vault-occupying portion configured to occupy at least a portion of a glenoid vault of the scapula and to couple with the bearing. The glenoid bearing support further includes a glenoid rim replacement portion attached to the glenoid vault-occupying portion. The interior surface of the glenoid rim replacement portion and the exterior wall of the glenoid vault-occupying portion defines a bone graft receptacle that is configured to receive a bone graft material.

US 2013/110470 discloses an implant for repairing a defect in an anatomical feature, such as by filling the volume of a defect in an articular surface of a glenoid fossa. The implant represents a final product after bone graft is shaped to match the surface of the defect and to form a continuous surface with the articular surface surrounding the defect. Thus, the implant includes at least two distinct surfaces, namely a first surface, which nests and fill the volume of the defect, and a second surface, which matches the surrounding articular surface.

### SUMMARY OF THE INVENTION

The present invention is joint prosthesis spacer as defined in claim 1.

A great improvement in BIO-RSA and other procedures that would benefit from adjusting lateral position of a component on a scapula would result if methods and systems could form and employ patient specific spacers. Patient specific spacers would better adapt to patient joint surface shape, for example at or around the glenoid surface of the scapula. Patient specific spacers can be formed anticipating that some reaming may occur, but that the glenoid will not be altered as extensively as in a conventional procedure. Patient specific spacers can be formed based on information about the condition and/or the shape of the joint surface of the patient to be augmented by a spacer. The patient information can be collected by a CT scan or by any other surface characterization technology. Thereafter, the patient information can be used as an input to shape a spacer from a bone graft material. The system and method can be used to shape any one or more of a section of bone from a proximal portion of the humerus, an allograft, a synthetic bone, or another structure. In a preferred embodiment the spacer is formed from a unitary piece of bone. The system and method can be used to form a plurality of pieces of bone or synthetic bone stock into a solid, unitary patient specific spacer.

### BRIEF DESCRIPTION OF THE DRAWINGS

Various embodiments are depicted in the accompanying drawings for illustrative purposes, and should in no way be interpreted as limiting the scope of the embodiments. Furthermore, various features of different disclosed embodiments can be combined to form additional embodiments, which are part of this disclosure.
Figure 1 is a partial cross-section of a shoulder joint, including portions of a scapula and a humerus as well as a reverse shoulder joint prosthesis components coupled therewith;
Figure 1A is a partial cross-section of a scapula having a glenoid component of an anatomical shoulder joint prosthesis coupled therewith;
Figure 2 illustrates a method of forming a patient specific insert negative, which can be used in a bone press, based on spatial location information;
Figure 3 illustrates a method of forming a patient specific bone graft, the graft being adapted for use in a joint replacement or repair procedure;
Figure 4 illustrates another method of forming a patient specific bone graft, the graft being adapted for use in a joint replacement or repair procedure;
Figure 5 is a perspective view of one example of a bone press;
Figure 6 is a cross-sectional view of the bone press of Figure 5 taken at section plane 6-6;
Figure 6A is a view similar as Figure 6, showing another example of a bone press;
Figure 7(A) is a partial exploded view of the bone press of Figure 5, with a bone graft blank, and a glenoid baseplate removed from the bone press;
Figure 7(B) is a partial exploded view of the bone press of Figure 5, with the glenoid baseplate and the bone graft blank disposed on a base of the bone press;
Figure 7(C) is a partial exploded view of the bone press of Figure 5, with a housing of the bone press disposed over and around the glenoid baseplate and the bone graft blank;
Figure 7(D) is an assembled view of the bone press of Figure 5, with a patient specific insert negative disposed on the bone graft blank and at least partially within a pressing zone of the bone press;
Figure 7(E) is an assembled view of the bone press of Figure 5 , with an actuator engaged with the patient specific insert negative prior to compressing the bone graft blank in the pressing zone;
Figure 7(F) shows a patient specific bone graft that can be formed in the bone press of Figure 5;
Figure 8(A) shows a perspective view of a first example of a patient specific insert negative;

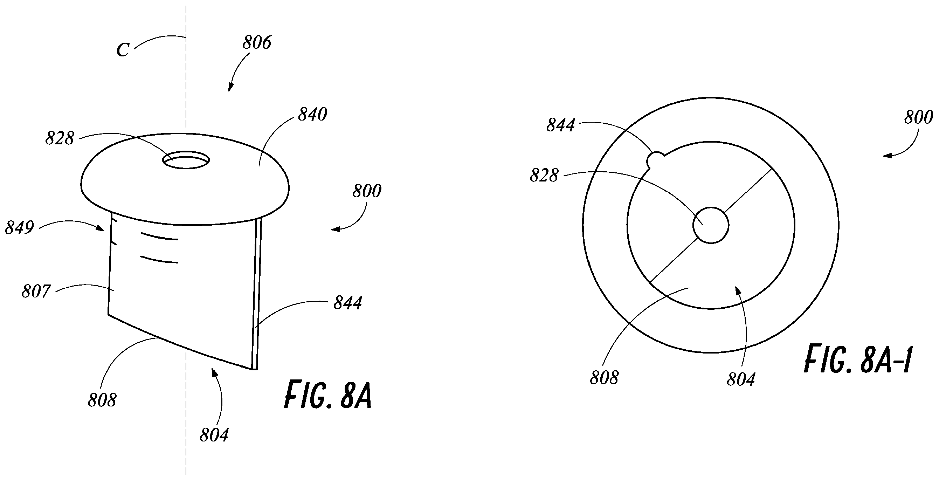

Figure 8(A)-1 is a bottom view of the patient specific insert negative shown in Figure 8(A);

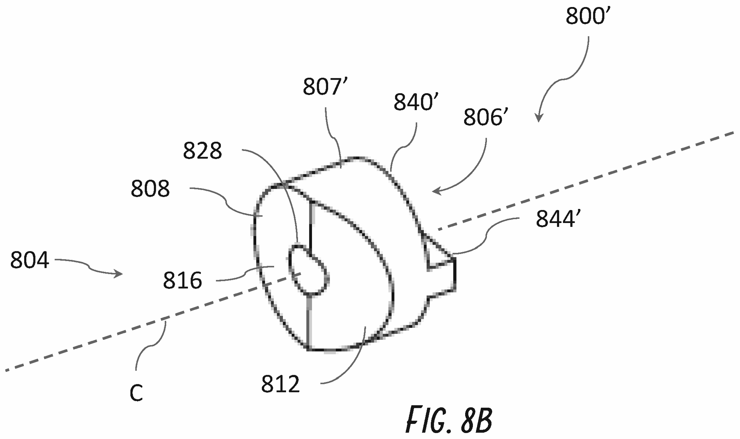

Figure 8(B) shows a perspective view of a second example of a patient specific insert negative;

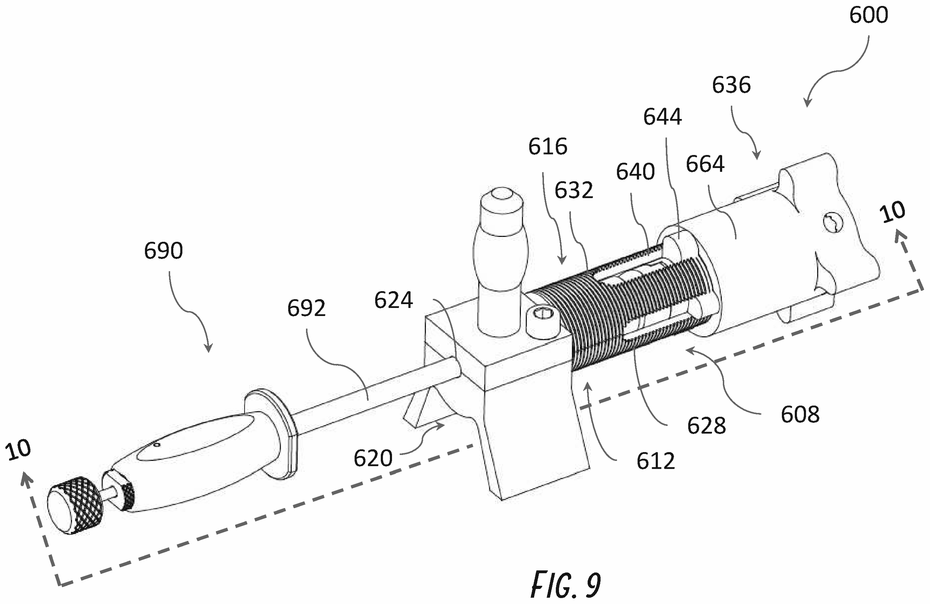

Figure 9 is a perspective view of a second example of a bone press in which an end of a baseplate impactor is disposed in a pressing zone of the bone press during the formation of the patient specific bone graft and in which compression being applied in the pressing zone by actuating a threaded coupling;

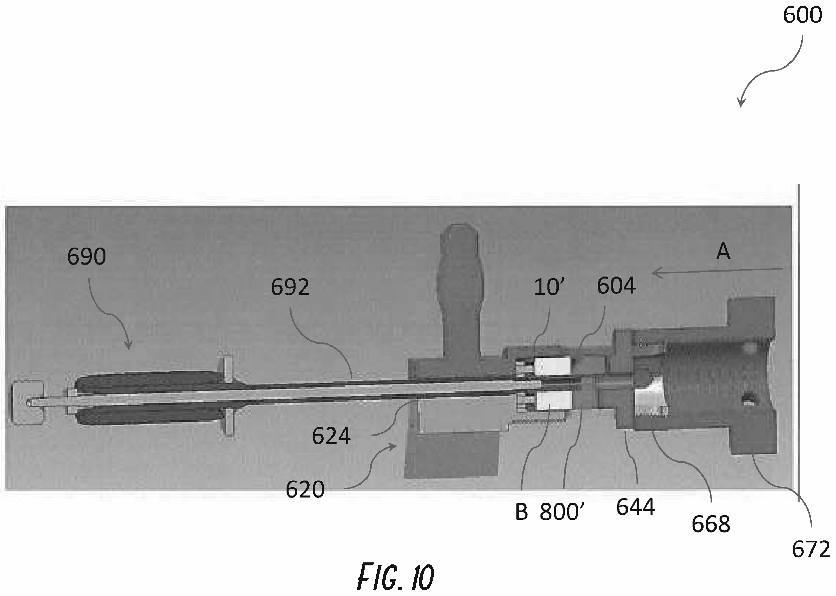

Figure 10 is a cross-section of the bone press of Figure 9 taken along the plane 10 - 10;

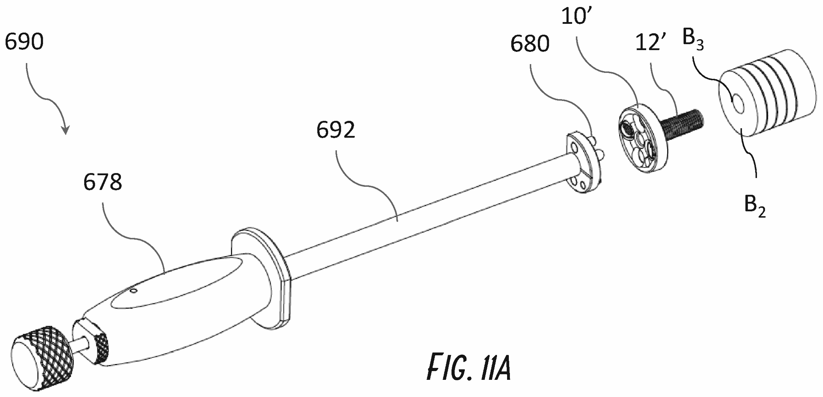

Figure 11(A) is a partial exploded view of an impactor, a joint prosthesis component, and a bone graft blank that can be placed in a bone press to form a patient specific bone graft on the impactor;

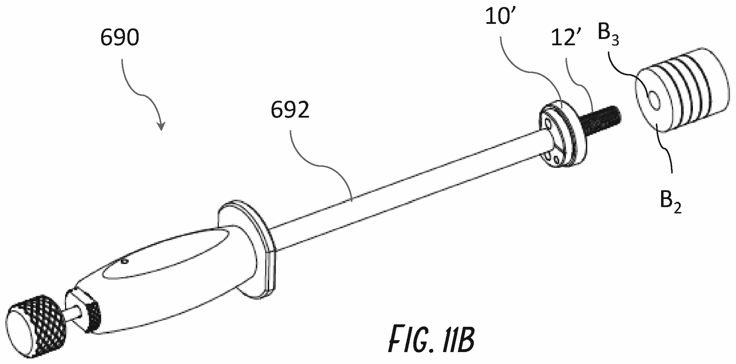

Figure 11(B) is a partial exploded view similar to that of Figure 11(A) showing the joint prosthesis component mounted on the impactor;

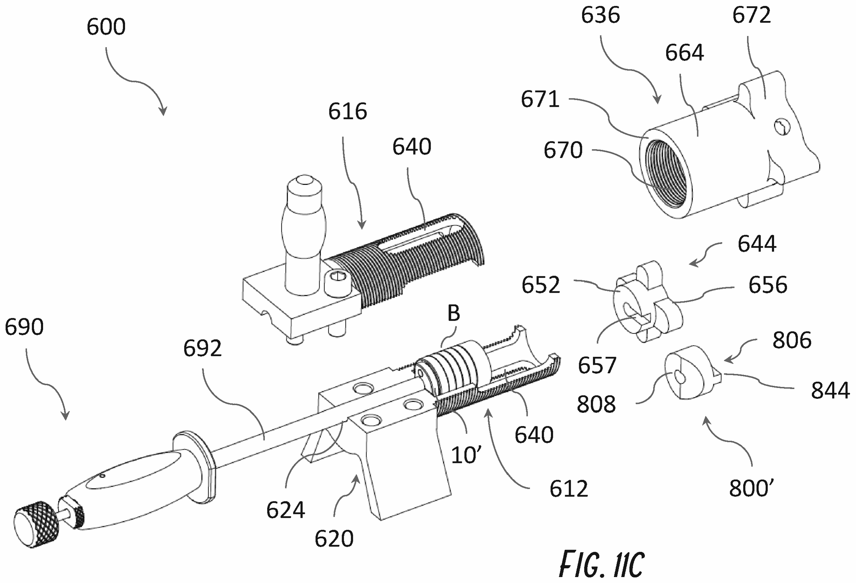

Figure 11(C) is a partial exploded view of the bone press of Figure 10 showing an end of an impactor, a joint prosthesis component, and a bone graft blank disposed on a surface of a structure partially forming a pressing zone;

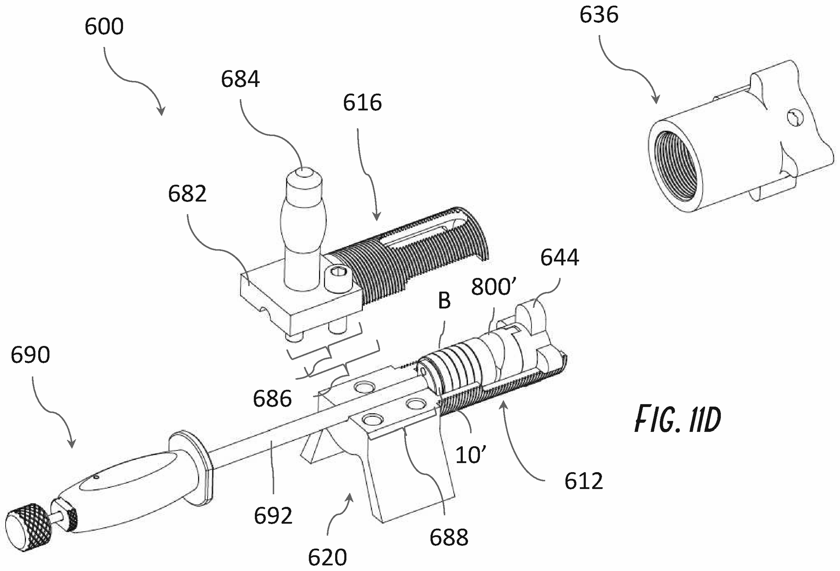

Figure 11(D) is a partial exploded view similar to that of Figure 11(C) showing a compression member placed on a lower shell of a base of the bone press, the lower shell partially forming a housing disposed around the pressing zone;

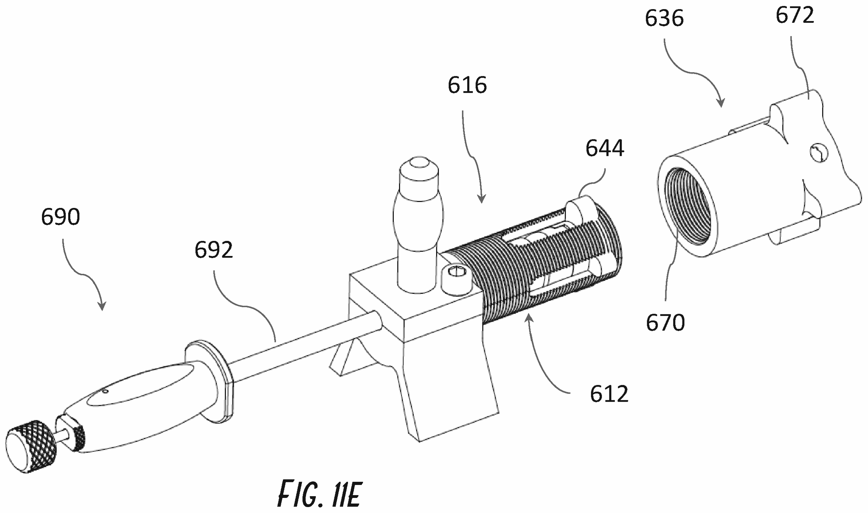

Figure 11(E) is a partial exploded view similar to Figure 11(C) showing an upper shell placed over the lower shell to form a housing disposed around the pressing zone;

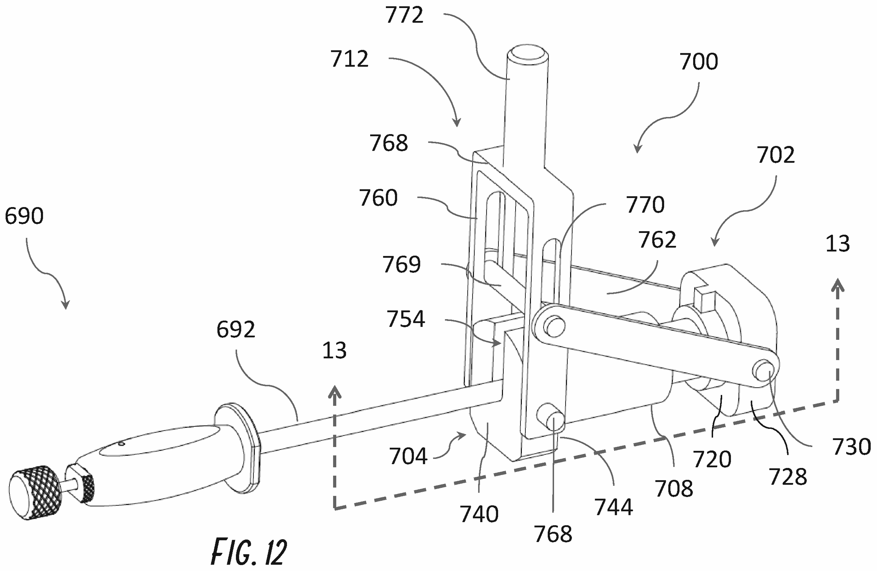

Figure 12 is a perspective view of a third example of a bone press in which a non-threaded actuator, e.g., a lever, applies compression in a pressing zone during the formation of a patient specific bone graft;

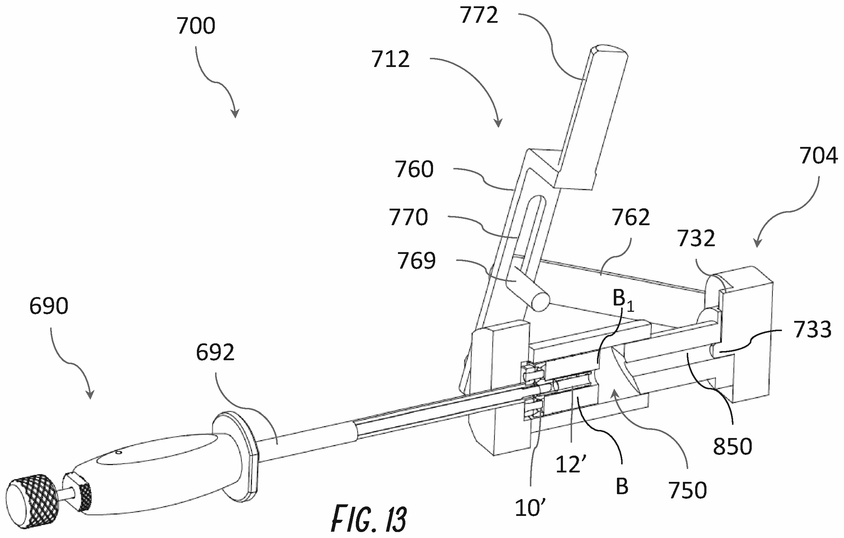

Figure 13 is partial cross-sectional view of the bone press of Figure 12 taken along section plane 13 - 13;

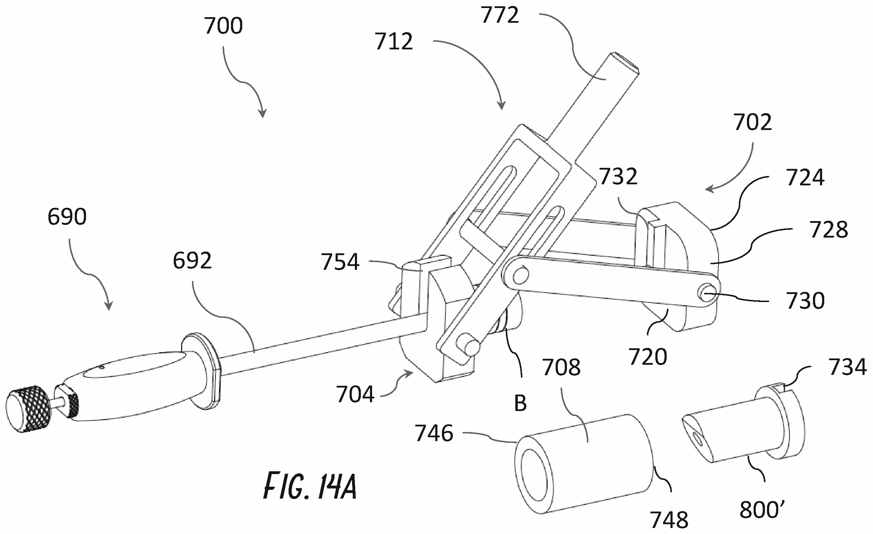

Figure 14(A) is a partial exploded view of the bone press of Figure 12 with an impactor, a joint prosthesis component, and a bone graft blank received in and on a base thereof; and

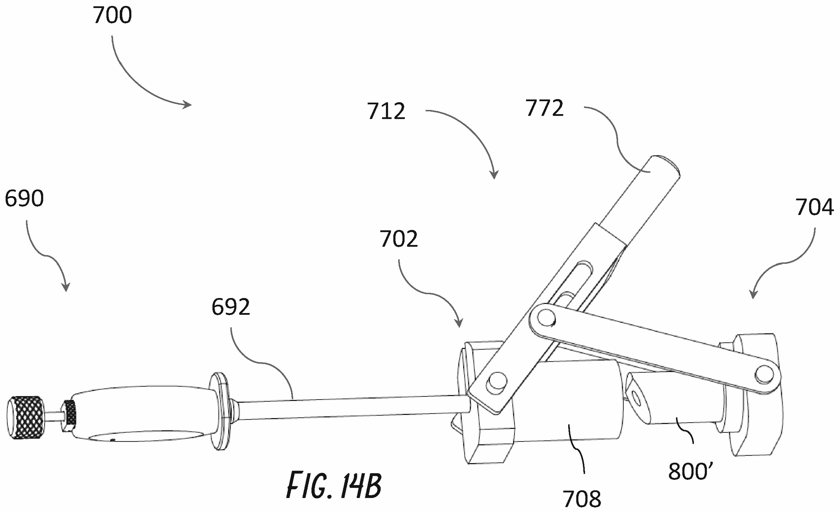

Figure 14(B) is an assembled view prior to actuation of a lever to apply compression in the pressing zone, with a housing disposed around the bone graft blank and the patient specific insert negative coupled with a compression plate.

### DETAILED DESCRIPTION OF THE PREFERRED EMBODIMENT

While the present description sets forth specific details of various embodiments, it will be appreciated that the description is illustrative only and should not be construed in any way as limiting. Furthermore, various applications of such embodiments and modifications thereto, which may occur to those who are skilled in the art, are also encompassed by the general concepts described herein. Each and every feature described herein, and each and every combination of two or more of such features, is included within the scope of the present invention provided that the features included in such a combination are not mutually inconsistent and are within the scope of the claims.

This application is directed to patient specific bone grafts, methods for forming and using, as well as apparatuses for forming, such grafts. As discussed above, patient specific grafts can be clinically useful in supplementing eroded, thin or weak bone to which a prosthesis component, such as a glenoid baseplate, is to be coupled. BIO-RSA is one procedure where such grafts can be used. The apparatuses and methods disclosed herein enable novel and less traumatic bone preserving joint replacement techniques. The apparatuses and methods disclosed herein can extend orthopedic treatments to patients who would otherwise not be treatable and can help preserve the possibility of future revisions if needed.

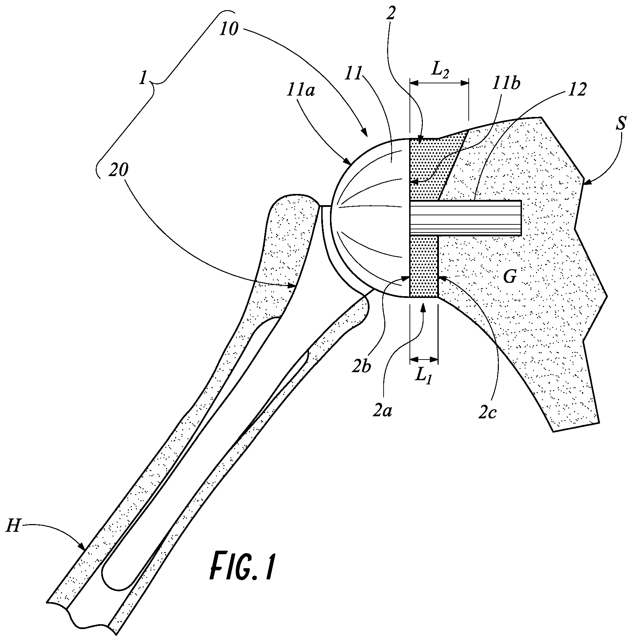

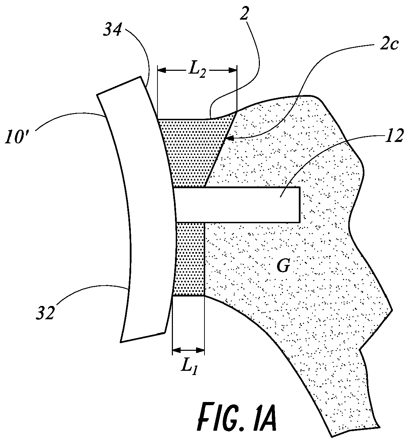

Figure 1 shows a shoulder prosthesis 1 comprising a glenoid component 10 implanted in the scapula S and a humeral component 20 implanted in the humerus H of a patient's shoulder. The glenoid component 10 has a head 11 which has, on a lateral side, a convex articular surface 11A of generally hemispherical shape. The lateral side is a side facing away from the glenoid surface G of the scapula S. The head 11 has an opposing face 11B disposed on a medial side. The medial side is a side facing toward the glenoid surface. In Figure 1, the face 11B is generally planar but the face 11B can have other shapes. Figure 1 illustrates what is sometimes referred to as a reverse should prosthesis because the position of the convex portion of the shoulder joint is the opposite of that of normal anatomy.

Figure 1A shows another approach to shoulder replacement where a component 10' having a concave surface 32 is mounted to the glenoid G. The component 10' is sometimes referred to herein as a baseplate, though a baseplate can include any structure of a prosthesis that directly contacts a side of a bone graft. The concave surface 32 is configured to receive a spherical member of a humeral component. An opposing face 34 of the component 10' is oriented toward the glenoid G. The surface 32 is on a lateral side of and the face 34 is on the medial side of the components 10'.

The glenoid components 10, 10' can also comprise an anchor member 12 that extends from the face 11B (or the face 34) in the direction away from the face 11A (or the surface 32). The free end of the anchor member 12 is securely anchored in the scapula S through the glenoid surface G when these components 10, 10' are implanted to join the components to the scapula S. By way of non-illustrated variation, the anchor member 12 can be externally threaded or, generally, have a surface state promoting bony ingrowth or other mode of anchoring. The anchor member 12 can be separable from or a unitary construction with other the component 10, 10'.

A bone graft 2 is positioned between the glenoid surface G and the face 11B, 34 of the respective glenoid components 10, 10'. The bone graft 2 has a periphery 2A and a lateral surface 2B. The lateral surface 2B is located on the side of the bone graft 2 disposed away from the glenoid surface G. The lateral surface 2B is covered by the face 11B of the head 11 or by the face 34 of the baseplate 10'. A medial surface 2C of the bone graft 2 faces and usually is in direct contact with the glenoid surface G. Once the bone graft 2 fuses with the glenoid surface G, the effective glenoid surface is displaced laterally outward to the distal surface 2B of the bone graft 2.

Figure 1 shows that the bone graft 2 can have a non-constant thickness. For example, a dimension L₁ at the inferior portion of the glenoid can be smaller than a dimension L₂ at a superior portion. Providing greater medial-lateral thickness at the superior portion can compensate for wear. Because the bone degradation or wear of every patient is different, the best thickness of the bone graft 2 for superior and inferior portions can be different for each given patient. Specific methods and apparatuses to discern the best thickness profile or other configuration of the bone graft 2 and to form the bone graft accordingly are discussed hereinbelow. More particularly, the medial face 2C preferably has a three-dimensional morphology that matches the three-dimensional morphology of, e.g., is a negative of, the glenoid or of the glenoid as the clinician intends to modify it prior to mounting the glenoid component 10, 10' to the scapula.

### Methods Related To Making Patient Specific Bone Grafts

A number of methods can be employed to make bone grafts for a joint procedure that are specific to a particular patient. These methods can be performed by a manufacturer of prostheses, by an imaging service provider, by a surgeon, or by a combination of an imaging service provider, a manufacturer, and a surgeon at the direction and under the control of the surgeon or other participants in these processes.

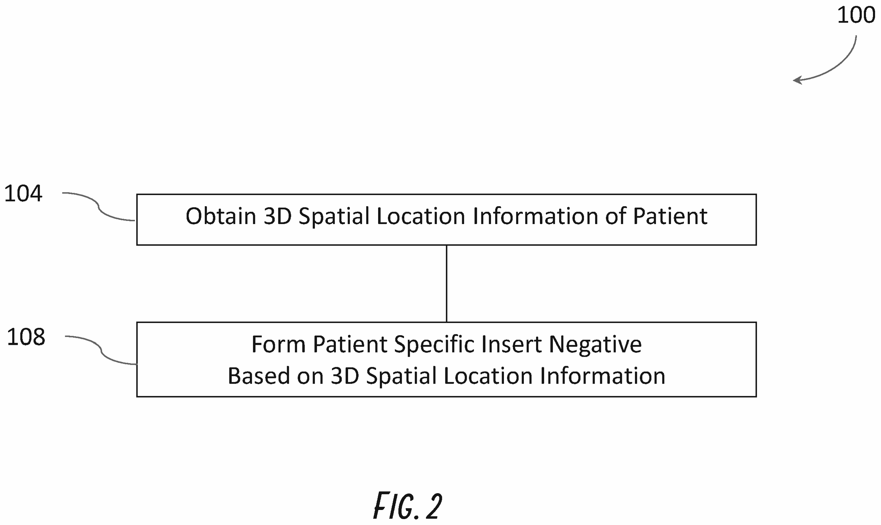

Figure 2 shows a method 100 for making a patient specific insert negative 800. Figure 8(A) shows an example of the patient specific insert negative 800. The patient specific insert negative 800 is referred to as a negative because, as discussed further below, a contoured surface 808 thereof has a shape that matches but is the opposite or is the negative of a shape to be formed on a bone graft blank B. The bone graft blank B is in turn a negative of a corresponding surface of the glenoid. The negative 800 is sometimes referred to as an "insert" herein because in certain approaches, the negative 800 is a separate component of a bone press and is inserted into the bone press prior to making the patient specific bone graft. This approach allows a bone press to be reusable while the negative 800 can be used for one specific procedure, then discarded as discussed below. However, as discussed further below, a patient specific bone press could be used, in which the contoured surface 808 of the negative 800 is integrated into and may not be inserted into nor removed from a bone press.

Figure 2 shows a method that can be performed by or at the direction of a manufacturer of prosthetic joint components. In a step 104 three dimensional spatial location information of a bone portion of a joint is obtained. Any suitable technology can be used to obtain the spatial location information. For example, one or more imaging technologies such as CT scanning, X-rays imaging or the like can be used to generate spatial location information. A mechanical device, such as a profilometer could be used to generate spatial location information without imaging. Three dimensional spatial location information can be obtained as one part of a broader process for characterizing the bone portion to determine how to treat the patient. For example, in the case of performing a shoulder replacement, a degree of retroversion may be determined to assess whether to perform an anatomical shoulder replacement (Figure 1A) or a reverse shoulder replacement (Figure 1).

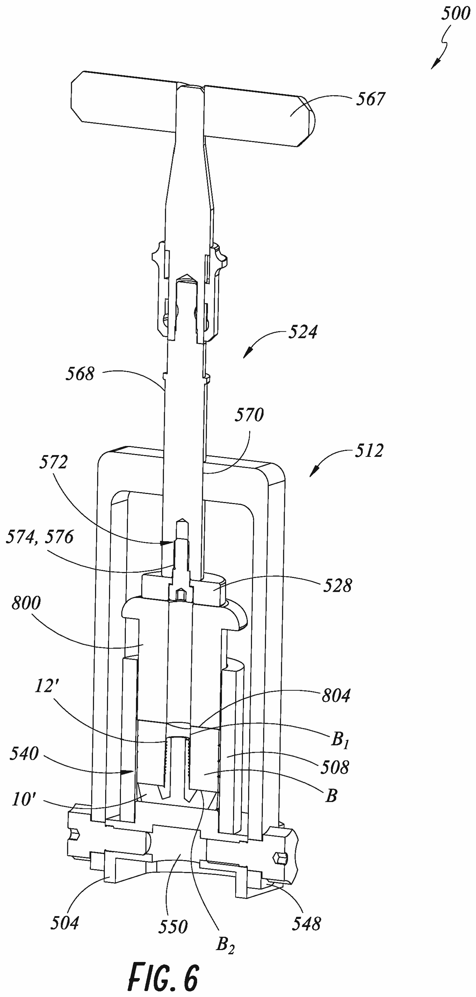

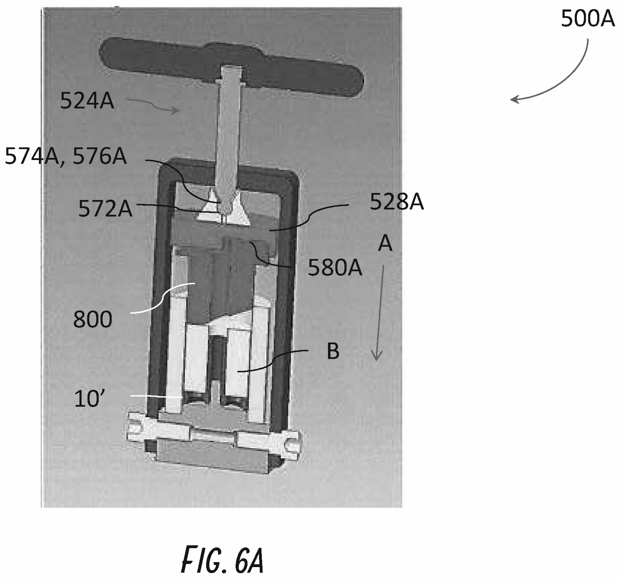

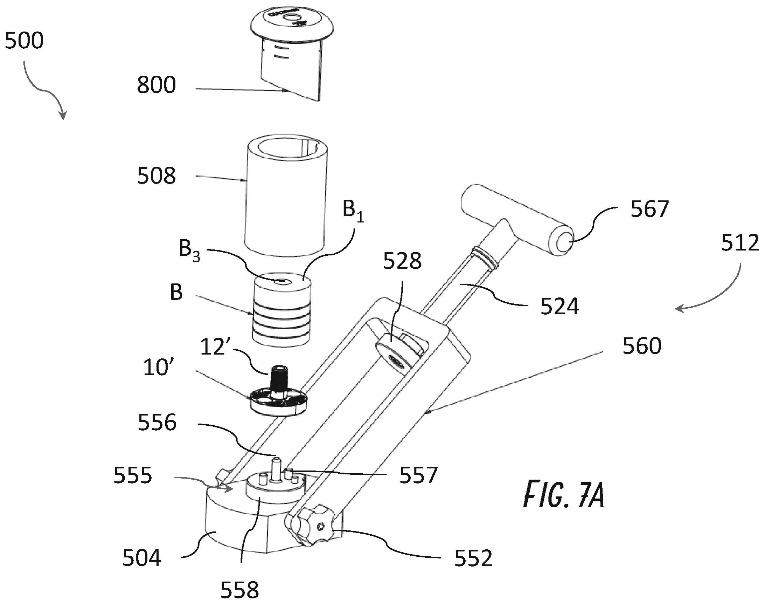

In a step 108, the patient specific insert negative 800 is formed. As discussed above, the negative 800 is configured with a contoured surface 808 to be an opposite of the bone graft to be formed in a bone press. The shape of the contoured surface 808 can be derived from the information concerning a bone portion that was characterized in the step 104. Therefore the step 108 can include shaping a first side 804 of the negative 800 based upon the three dimensional information. The patient specific insert negative 800 is configured to be advanced into engagement with a side of a bone graft blank B as shown in Figures 6 and 7(A). As discussed in more detail below, the bone graft blank B includes a volume, e.g., a cylinder or a plurality of pieces, of bone matter that does not necessarily match the shape of the glenoid surface or other bone portion analyzed in connection with step 104. In certain methods, the shape of the bone graft blank B is altered by being compressed against the negative 800 in a bone press. The negative 800 thus must be sufficiently stiff to retain the shape that is based upon the three dimensional information obtained in the step 104.

Figures 8(A) and 8(B) show examples of patient specific insert negatives 800, 800'. The insert negatives 800, 800' includes the first side 804 configured to change a generic shape of a surface of the bone graft blank B or other bone material into a patient specific shape as discussed further below. The insert negatives 800, 800' also include a second side 806, 806' opposite the first side 804. The insert negative 800, 800' also includes a peripheral surface 807 that extends between the first side 804 and the second side 806, 806'. The first side 804 can includes a contoured surface 808. The contoured surface 808 can have a first portion 812 that corresponds to a portion of bone to be augmented and a second portion 816 (best seen in Figure 8(B), but corresponding surfaces could be provided in the embodiment of Figure 8(A)). The second portion 816 can be configured to be mated with a portion of a bone that is to be augmented differently. The second portion 816 can be configured to be mated with a portion of a bone that is to be augmented by a lesser amount than the portion to which the first portion 812 of the contoured surface 808 is to be mated to.

As discussed above, the surface 804 is an opposite of or a negative of the surface to be created on the bone graft blank B. Accordingly, the second portion 816 which is configured to form the zone to provide lesser augmentation on the bone graft blank B extends away from the second side 806, 806' by a first amount. The first portion 812 which is configured to form a zone of greater augmentation on the bone graft blank B extends away from the second side 806, 806' by a second amount less than the first amount which is provided in the second portion 816. In one example, the second portion 816 comprises a planar surface disposed in a plane that is perpendicular to a direction of compression C in a bone press. The direction of compression C can be generally along an axis extending through a central aperture 828 of the patient specific insert negative 800, 800'. The first portion 812 can be a planar portion that is disposed along a plane that is oriented at an acute angle to the axis C. The second portion 816 can be inclined toward the second side 806. The first portion 812 can have a boundary contiguous with the second portion 816 and can be spaced away from the plane of the second portion at locations away from the contiguous boundary. An advantage of providing two planar portions is that it enables the surgeon to perform two reaming steps to create two planar bone surfaces to which planar surfaces of a patient specific bone graft formed by the negative 800 can be mated.

One skilled in the art will recognize that more complex shapes can be formed by forming the first and second portion 812, 816 with complex geometries or by providing more portions on the first side 804 resulting in a more complex contoured surface 808. The advantages of more complex contoured surfaces include allowing the patient specific bone graft to more closely conform to the patient's natural anatomy. This can allow the surgeon to modify the bone to a lesser extent or even to just apply the patient specific bone graft to the glenoid without much or any modification.

To achieve more complex configurations, the contoured surface 808 can be based on three dimensional spatial location information obtained in the step 104 of Figure 2. The patient specific insert negative 800 is configured to be mounted on or in a bone press, for example as described below. The bone press and the patient specific insert negative 800, 800' cooperate to shape the volume of bone material, which can be formed as a bone graft blank, as discussed below, upon application of pressure in the bone press.

The second side 806' is configured to mate with a bone press as discussed in greater detail below in connection with Figures 9-11E. The second side 806, 806' can have a surface 840 for engaging a surface of a compression plate 644 of the bone press 600. The compression plate 644 can include a surface oriented perpendicular to a direction of advance of the compression plate 644 in the press 600, which generally corresponds to the axis C. The second side 806' can also be configured with a keyed projection 844'. The keyed projection 844' can be configured to be received in a slot 657 of the bone press 600 of Figures 9-11E or in a slot 580A of the compression plate 528A of the bone press 500A of Figure 6A. A keyed interface between the keyed projection 844 and the slot 657 (see Figure 11(C)) provides a secured interface between the negative 800 and the bone press 600. The projection 844 and the slot 657 retain the insert negative 800 in a fixed rotational position. The fixed rotational position prevents rotation of the first surface 804 relative to an opposing surface of the bone graft blank B as discussed further below.

The second side 806 of the insert negative 800 can include a generally flat surface 840 although it may be rounded toward the periphery. In one embodiment, the insert negative 800 includes a keyed projection 844 that extends along the peripheral surface 807. The projection 844 can extend laterally into a longitudinal channel 580 discussed in more detail below. The peripheral surface 807 can include indicia 849 that are useful in confirming a degree of compression of the bone graft blank B as discussed further below. The indicia 849 are patient specific in that they may provide a relationship between the surfaces 812, 816 and a degree of compression due to movement of the surfaces in a bone press as discussed further below.

Further features that are convenient for integrating the patient specific bone insert negative 800 into a bone press are discussed below in connection with the bone press 600.

The method of Figure 2 can advantageously be performed in a number of different settings. For example, a special facility can be used to form the patient specific insert negative 800. The insert negative 800 will be subject to high compression, e.g., pressures and forces, in some cases. The insert negative 800 will be formed of a material that will not yield under such conditions. Suitable materials include one or more of stainless steel, maraging steel, cobalt chromium, inconel, nickel alloys, aluminum, titanium, PEEK, and other polymers.

In one process flow, step 104 is performed at an imaging center that can be run by the surgeon or at a separate facility. Data generated in the step 104 can be transmitted by any means to a manufacturing facility that can create the insert negatives. In another approach, the step 108 can be performed with equipment that could be located in a factory or in a surgeon's facility. For example, direct laser metal sintering and other three-dimensional printing technologies could be adapted to be deployed in either setting to form the patient specific insert negative 800 in the step 108.

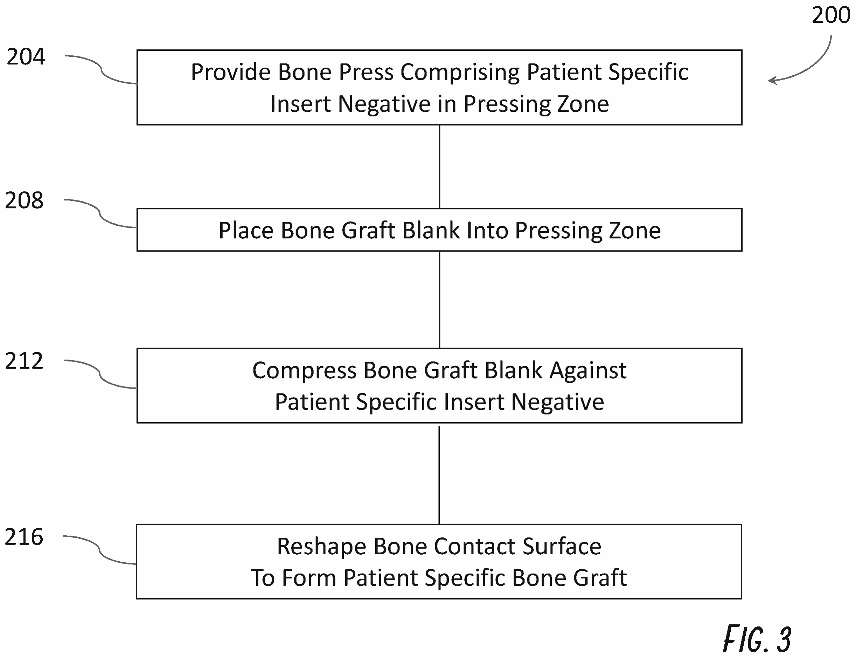

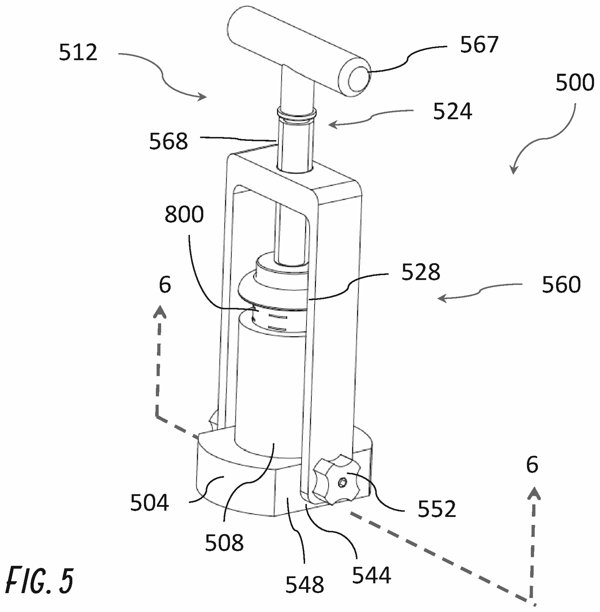

Figure 3 illustrates another method 200 that can be performed by an implant manufacturer or a surgeon. The method 200 will be discussed in connection with certain features of a bone press 500, shown in Figures 5-7E. However any other bone press compatible with the method could be provided, including any of the bone presses described herein. In one approach, the method 200 is performed by the surgeon or surgical staff or under a supply agreement between the surgeon and a third party.

In a step 204 the bone press 500 is provided. The bone press 500 is provided with the patient specific insert negative 800. The patient specific insert negative 800 can be produced by the method 100 or otherwise. The negative 800 can be produced by a third party and supplied to the party performing the method 200.

The patient specific insert negative 800 can be provided in a pressing zone 540 of the bone press 500. The pressing zone 540 includes a volume that is contained within structures of the bone press 500. For example, as discussed below the bone press 500 includes a base 504, a housing 508, and an actuator 512. The pressing zone 540 is defined in the bone press 500 between ends thereof by the base 504 and the patient specific insert negative 800. An outer bound of the pressing zone 540 is defined by an inside surface 510 of the housing 508. The housing 508 can be a solid cylindrical sleeve. In some embodiments, the housing 508 is clear or has a clear portion such that compression of the bone graft blank B therein can be observed by the user. The housing 508 can have an inner diameter that is about the same dimension as an outer diameter of a cylindrical bone graft blank B. In one example, the bone graft blank B is about 25mm in diameter and the inner diameter of the housing 508 is about 25 mm. In another example, the bone graft blank is about 25mm in diameter and the inner diameter of the housing 508 is about 26, about 27, about 28, about 29, or about 30 mm. In another embodiment, the inner diameter of the housing 508 can be 20-30% larger than the diameter of the bone graft blank B.

The housing 508 could be formed with indicia instead of or in addition to the insert negative 800 to provide feedback to the user as to the degree of compression of a bone graft blank in the bone press 500.

In step 208 a bone graft blank B is placed in the pressing zone 540. Although the bone graft blank B can be placed directly on the base 504, in certain variants of the method another structure can be placed on the base 504 and that other structure can define an end or a portion of the pressing zone 540.

Figures 5-7E illustrate methods when the glenoid component 10' optionally is placed on the base 504. In a modified embodiment, the methods ofFigures 5-7E can be performed without the glenoid component 10' placed on the base 504, e.g., with a bone graft blank placed directly on the base 504. The glenoid component 10' defines a portion of the pressing zone 540. In particular, the medial face 34 of the glenoid component 10' can form an end of the pressing zone 540. As discussed above, the glenoid component 10' has anchor member 12'. The opposing face 32 provides an end of the pressing zone 540. In one variation of the method 200, the anchor member 12' is disposed in the pressing zone 540. The step 208 can involve placing a cylindrical bone graft blank B over the anchor member 12'. In this variation, the anchor member 12' is radially inward of and is surrounded by the bone graft blank B. In this variation, the bone graft blank B is radially inward of and is surrounded by the housing 508. The pressing zone 540, e.g., the space where compression of the bone graft blank B occurs, can be at least partially defined between the anchor member 12' and the housing 508.

The method 200 can continue with a step 212 in which the bone graft blank B is placed under compression. The compression can be due to pressure being applied to medial and lateral ends B_{1,} B₂ of the bone graft blank B. The compression can be due to a force being applied to medial and lateral ends B_{1,} B₂ of the bone graft blank B. Compression can be applied in the pressing zone 540 by providing relative motion between the patient specific insert negative 800 and the base 504 or a portion of the base 504 or of a component coupled with the base.

The step 212 can involve actuating the patient specific insert negative 800 and thereby moving the negative 800 toward the cover 504 as indicated by an arrow A in Figure 6. In the bone press 500, the patient specific insert negative 800 is moved toward the base 504 by the actuator 512. The patient specific insert negative 800 is moved by rotating a screw member or ram 524 to advance a compression plate 528 toward the base 504. The compression plate 528 can include a rigid plate configured to couple with the patient specific insert negative 800.

Other structures that could be used to move the patient specific insert negative 800 into compression with the bone graft blank B can include a lever arm (discussed below in connection with 12-14B), a pneumatic mechanism, or other similar mechanisms. The actuator 512 can be hand operated or automatically driven. The actuator 512 can have a handle with an arcuate form between lateral ends thereof to conform to the shape of the palm of the hand of a user.

In a step 216, the bone graft blank B is reshaped to form a patient specific bone graft. For example, the compression provided in the step 212 can be continued or increased until ends of the bone graft blank B change their shape in a suitable manner. The reshaping can involve shortening the bone graft blank along the axis C by 25-75 %, by 35-65 %, for example by about 50 %. The reshaping can include modifying a planar or irregular medial side or end B₁ of the bone graft blank B to have a specified profile. For example, as discussed above the first side 804 of the insert negative 800 can have a profile that matches a glenoid shape or a shape to which the glenoid will be modified in a surgical procedure. In one example, the glenoid is to be reamed to planar faces that are disposed along a contiguous boundary but angled to one another. In such case, the insert negative 800 is formed to have a shape that matches that of the glenoid or the shape to which the glenoid is to be modified. In the step 216, the insert negative 800 can have two angled faces on the medial side as shown in Figure 8(B). The compression can be continued or increased by continuing to move the insert negative 800 toward the base 504 after initial contact is made between the insert negative 800 and the bone graft blank B. Such movement can continue for 10-20 mm or for up to about 50% or even as much as 60%, 65%, or even 70% of the initial uncompressed length of the bone graft blank B. The step 216 causes the bone graft blank B to be compressed and to conform to a surface of the negative 800 and generally to irreversibly deform so as to hold the shape once compression is removed. This is not an elastic deformation only where the bone springs back to its initial shape once compression is removed, though some limited recoil may occur.

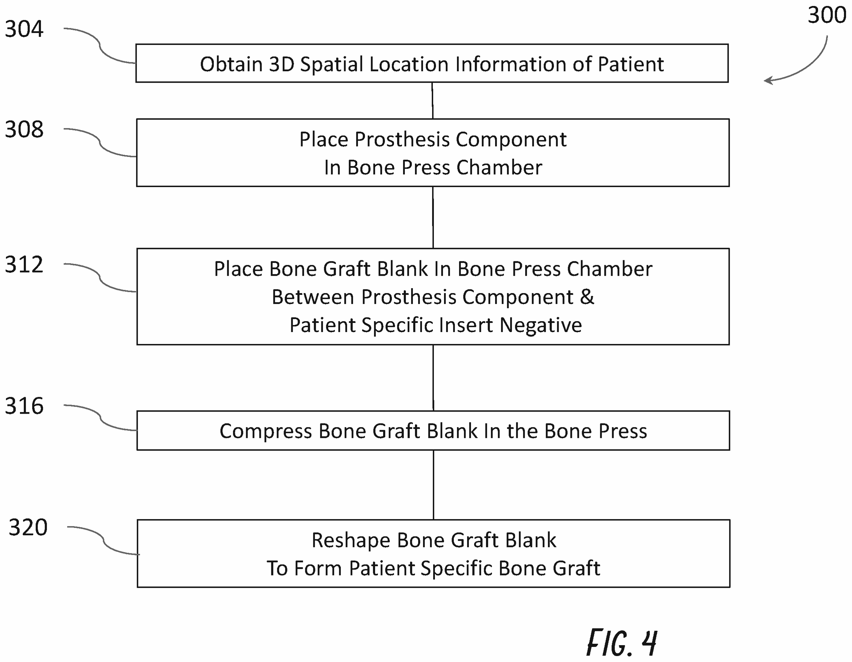

Figure 4 illustrates a method 300 in which three dimensional spatial location information is obtained and a patient specific bone graft is produced. This method can be conducted by or under the direction of a surgeon or by or under the direction of a third party, such as an implant manufacturer. The method 300 includes a step 304 in which three dimensional spatial location information is obtained. The step 304 can be similar to the step 104, such as including the conducting of a CT scan or x-ray of a relevant portion of a patient's bone or joint.

In a step 308 a prosthesis component, such as the component 10', optionally is placed in a bone press. With reference to Figure 7A, the component 10' can be placed directly onto the base 504 of the bone press 500. In another variation, the component 10' can be placed on an impactor 690. Thereafter, a portion of the impactor 690 and the prosthesis component can be placed in the bone press together. In another variation, the component 10' is not placed in the bone press 500 during a method of forming a patient specific bone graft.

In a step 312, a bone graft blank, such as blank B, is placed in the bone press 500. Figure 6 shows that the bone graft blank B can be placed in the bone press 500 in a location between the base 504 and the patient specific insert negative 800. In one method, an end or lateral side B₂ of the bone graft blank B faces toward the base 504 and an end or medial side B₁ faces toward the patient specific insert negative 800. As discussed above, the bone graft blank B can take any suitable form. In some cases the bone graft blank B is a cylinder of bone with the sides B_{1,} B₂ being generally flat opposing sides and having a generally circular body extending therebetween. The bone graft blank B can be obtained from a resected portion of the humerus or from another bone segment. As a result, in some cases, the bone graft blank B can be more irregularly shaped as a result of being cut during a procedure from the patient undergoing shoulder replacement. The bone graft blank B also can include two or more pieces that are placed into the housing 508 and later compressed into a single graft.

In one technique, the prosthesis component 10' with the bone graft blank B mounted thereon is placed in a bone press, such as the press 500. As shown in Figure 7(A), for example, the component 10', which has an anchor member 12', can have a central lumen B₃ of bone graft blank B placed thereover. The lumen B₃ can be sized to permit the bone graft blank B to be slipped over the anchor member 12'. So, in one variation the step 312 includes providing relative motion between the anchor member 12' of the component 10' and the bone graft blank B such that the anchor member 12' is moved into the lumen B₃. After the bone graft blank B is mounted on the prosthesis component 10', the component and the blank can be placed into the bone press 500. In a variation, the prosthesis component 10' is not placed in the bone press 500. The blank B can be placed directly onto the base 504 or another component of the bone press 500.

Figure 6 shows that when the bone graft blank B is placed in the bone press 500 the medial side B₁ is adjacent to the patient specific insert negative 800. In one approach no components are placed between the medial side B₁ and the first side 804 of the negative 800. Direct contact can be provided between the medial side B₁ and the first side 804 of the negative 800. Direct contact allows the surface profile of the medial side B₁ and the first side 804 to be provided on the bone graft blank B as discussed further below.

In a step 316 the bone graft blank B is compressed in the bone press. Figure 6 shows that the bone graft blank B is compressed in the pressing zone 540 between the patient specific insert negative 800 and the component 10'. More generally, the bone graft blank B is compressed between the patient specific insert negative 800 and the base 504. The compression provided in the step 316 is initially axial compression. Axial compression is along the direction of the arrow A (Figure 10). After an initial level of compression, the bone graft blank B may expand transverse to the direction A to some extent. However, the pressing zone 540 of the bone press 500 is enclosed by the housing 508. So expansion transverse to the lumen B₃ is limited. The bone graft blank B is disposed between the anchor member 12' and the housing 508. This configuration can provide compression in the transverse direction.

In a step 320 the bone graft blank B is reshaped. The step 320 can involve changing the length of the body of the bone graft blank B from a first length to a second shorter length, as discussed above in connection with the method of Figures 3. The step 320 can involve increasing the outer profile of the bone graft blank B, for example from a first radius to a second larger radius. If the shape of the medial side B₁ of the bone graft blank B has not been modified upon compression in the step 316, the step 320 can involve changing the side B₁ to a shape matching, but a negative of, that of the bone to which the bone graft blank B will be mated after it is changed into a patient specific bone graft. The step 320 causes the bone graft blank B to be compressed and conformed and generally to irreversibly deformed so as to hold the shape once compression is removed. This is not an elastic deformation only where the bone springs back to its initial shape once compression is removed, though some limited recoil may occur.

Following the method of Figures 3 and 4 the bone graft that is shaped in the bone press 500 (or other bone press) can be removed. If the initial diameter of the bone graft blank B is similar to the inner diameter of the housing 508 an ejector tool can be used to push the bone graft out of the housing 508. The ejector can include an elongate solid rod with a handle at one end and a disk or other pushing plate at the other end. The disk or pushing plate is configured to mate with and push the bone graft out of the housing 508. A kit including the bone press 500 (or other bone presses disclosed or claimed herein) can include an ejector.

### Bone Graft Presses

Turning now to Figures 5-14B a variety of bone graft presses will be discussed in greater detail. The bone graft presses of these embodiments can be hand operated. Figures 5 and 9 illustrate bone presses in which an actuator is provided that includes a threaded connection. Figure 12 shows a bone press that includes a single motion actuator, e.g., a non-threaded actuator, e.g., an actuator with a lever, to provide compression of a bone graft blank B. The bone graft presses can be used to form a patient specific bone graft directly on a component to which the bone graft is to be coupled. Figures 5-7E illustrate bone presses that form a patient specific bone graft directly on a glenoid baseplate to be coupled with an impactor or other surgical tool after the patient specific bone graft is formed. Figures 9-14(B) show a bone press that permits an impactor to be coupled with a glenoid baseplate prior to the formation of the bone graft in the bone press such that the baseplate and the bone graft are ready to be implanted immediately after the bone graft is removed from the bone press.

Figures 5-7E illustrate the bone press 500 in greater detail. The bone press 500 is configured to receive the bone graft blank B and the prosthesis component 10' together in the pressing zone 540. In other embodiments the bone graft blank B could be placed the pressing zone 540 without the component 10'. The actuator 512 of the bone press 500 includes a threaded mechanism for advancing the actuator 512 toward the base 504 which allows for a large amount of compression, if desired.

Figure 5 shows that the base 504 can comprise a plate-like configuration. That is, a lower surface 544 can be planar in shape allowing the base 504 to rest on a table or other flat surface in use. The lower surface 544 would be the bottom of the bone press 500 in such arrangements with an upper part of the actuator 512 being the top of the bone press. The bone press also can include two side surfaces 548 to which the actuator 512 is coupled. The side surfaces 548 can include threaded apertures 550 to which threaded members 552 of the actuator 512 can be coupled (see Figure 6). In one embodiment the threaded apertures 550 comprise end portions of one elongate lumen that extends from one of the surfaces 548 to the other of the surfaces 548.

Figure 7(A) shows that an upper surface of the base 504 can include a platform 555 configured to couple with the component 10' or other joint component. The platform 555 preferably allows the component 10' to be quickly coupled with the platform 555 to prevent rotation of the component 10' relative to the platform 555. For example a central post 556 can be provided that can be received into an aperture of the component 10'. One or more peripheral posts 557 can be provided spaced apart from the central post 556. The peripheral posts 557 also are received in apertures of the component 10'. The peripheral posts 557 prevent any torque that may be induced in the pressing zone 540 from resulting in rotation of the component 10' or from transmission of torque transferred between the upper surface 554 and the component 10' from being applied to the bone graft blank B. Providing isolation from some or substantially all such torque can be important if the material of the bone graft blank B is fragile because the goal of the bone press is to output a solid patient specific bone graft that will not crumble in subsequent using during a joint implantation procedure. The platform 555 can also be a locally elevated portion of the upper surface of the base 504. For example, upward cylindrical projection 558 can be provided on the upper surface of the base 504. The projection 558 allows the component 10' to come to rest at an elevation above a portion of the upper surface 554 located outward of the projection 558. The projection 558 can have a circular periphery with an outer radius less than the inner diameter of the housing 508 such that the housing can be advanced over the projection 558 and come into contact with the upper surface 554 at a location outward of the projection 558. This configuration allows the entire platform 555 to be received in the housing 508 during the compression of the bone graft blank B.

Figures 5 and 6 show the actuator 512 in more detail. The actuator 512 includes a frame 559 that provides a rigid connection to the base 504. In one embodiment the frame 559 includes two bars 560 that are pivotally connected at a first end 562 to the base 504 by the threaded members 552. The bars 560 include an elongate rigid portion that extends from the first end 562 to a second portion 564 at which the bars 560 are linked. The second portions 564 can be linked by a transverse bar 566. In some configurations, the bars 560 and the transverse bar 566 are portions of a unitary construction, e.g., they can be monolithic, they can be a single piece, and/or they can be seamless without boundaries between them. The bars 560 and the bar 566 (if present) transfer forces between the base 504 and a compression plate 528 of the actuator 512. The compression plate 528 is advanced by action of a handle 567.

Figures 5 and 6 show that the compression plate 528 can be placed between the handle 567 and the base 504. A ram 524 is coupled at one end with the compression plate 528 and at an opposite end with the handle 567. The ram 524 is configured to be advanced relative to the transverse bar 566, for example by a threaded connection. The ram 524 can have a threaded external surface 568 that mates with internal threads of a hole 570 formed in the inside surface of a hole formed in the transverse bar 566.

The connection between the handle 567 and the ram 524 can be any suitable connection. A suitable connection will transfer torque from the handle 567 to the ram 524 such that rotation of the handle is transferred directly into rotation of the ram. Such connection will generally prevent any relative rotation between the handle and the ram. The handle 567 can be directly coupled to the ram 524, such as by a pin or screw to assure one-to-one rotation between these components. The handle 567 and the ram 524 could be formed as a single component, e.g., be unitary in some configurations. The connection between the ram 524 and the compression plate 528 preferably is one which advancement of the ram 524 toward the base 504 is transferred one-to-one to the compression plate. The compression of the bone graft blank B that is preferred is primarily or substantially only in the axial direction. In this context, the axial direction is a direction defined along the central axis of the lumen B₃. The axial direction also can be defined as perpendicular to one or both of the surfaces B₁, B₂ of the bone graft blank B. To provide no or substantially no circumferential compression of the bone graft blank B the compression plate 528 is configured to remain rotationally fixed during advancement of the compression plate. Relative rotation of the compression plate 528 is provided by the connection between the plate 528 and the ram 524.

Figure 6 shows one mechanism for providing relative rotation between the compression plate 528 and the ram 524. In particular, a coupler 572 can be provided between an upper side of the compression plate 528 and the ram 524. The coupler 572 can include a stepped cylindrical recess 574 formed in the ram 524. The recess 574 can more generally be an arcuate chamber in some embodiments. The lower end of the ram 524 can receive a pin or axle with an arcuate surface 576 defined thereon. The arcuate surface 576 can optionally be stepped and preferably has a slip fit relative to the recess 574. The coupling of the cylindrical recess 574 and the arcuate surface 576 provide for rotation of the compression plate 528 relative to the ram 524. Although shown as a pin and recess connection, the connection could be one that provides at least one additional axis of rotation

Figure 6A shows another embodiment of a bone press 500A that is similar to the bone press 500 except as described or illustrated differently. The bone press 500A has a mechanism for providing relative rotation between a compression plate 528A and a ram 524A. In particular, a coupler 572A can be provided on an upper side of the compression plate 528A. The coupler can have an arcuate chamber 574A defined therein. The arcuate chamber 574A is at least partially spherical in one example. The lower end of the ram 524A can have an arcuate surface 576A defined thereon. The arcuate surface 576A can comprise an at least partially spherical surface. The coupling of the arcuate chamber 574A and the arcuate surface 576A provide for relative rotation of the compression plate 528A relative to the ram 524A. Although shown as a ball and socket connection, the connection could be one that provides relative rotation about fewer axes, e.g., about two axes. Also, the compression plate 528A can have a keyed arrangement formed thereon, as discussed further below.

Figures 7(A)-7(E) show how the bone press 500 is used. In Figure 7(A), the ram 524 and the compression plate 528 are raised by actuating the handle 567 to a position spaced away from the base 504. The plate 528 can be moved as far away from the base 504 as possible, in a position that may be described as fully retracted. The threaded members 552 can be loosened if necessary to permit the actuator 512 to be moved away from the platform 555 to allow access to the platform. The component 10' or other similar component can then be placed on the platform 555. Apertures in the component can be placed over the central and peripheral posts 556, 557. In this position the component 10' is secured to the platform 555 against undesirable rotation. The base 504 is rigid and holds the component 10' against axial movement away from the compression plate 528.

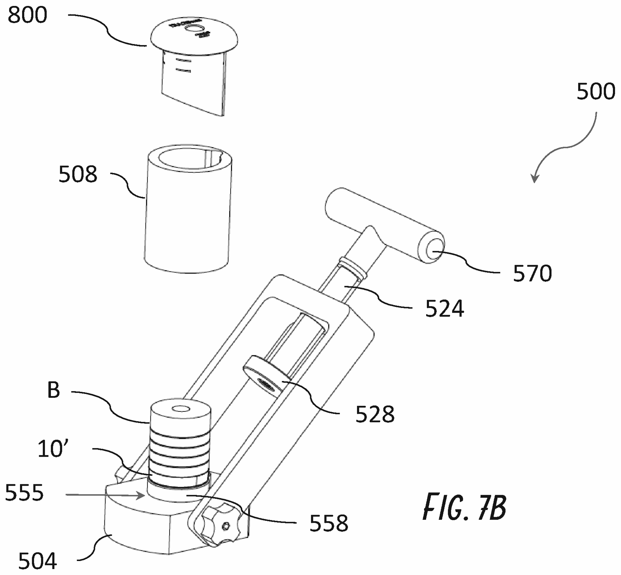

Figure 7(B) shows that the bone graft blank B can thereafter by placed over the component 10'. For example, the lumen B₃ of the bone graft blank B can be advanced over the anchor member 12'. The anchor member 12' can be received in the lumen B₃. The lateral surface B₂ of the bone graft blank B can be placed directly on the medial side of the component 10'. Figure 7(B) shows the component 10' and the bone graft blank B on the base 504 in a zone where the pressing zone 540 will be formed. The component 10' and the bone graft blank B are disposed on the projection 558 at an elevation above the surrounding areas of the platform 555.

Figure 7(B) shows that the housing 508 and the patient specific insert negative 800 are spaced apart from the rest of the bone press 500 at this point of one method. These components could be at the surgical table or on a back table in the operating room or could be elsewhere if the bone graft blank is formed well before the surgery.

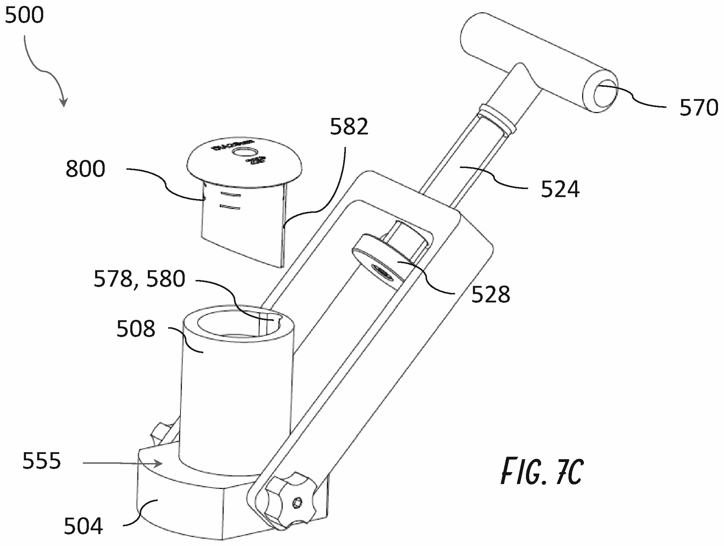

Figure 7(C) shows a later step in which the housing 508 is advanced over the bone graft blank B. The housing 508 can be advanced over the bone graft blank B and over the component 10' if present. In the illustrated method, the housing 508 is advanced over the blank B, the component 10' and over the projection 558 into contact with a surrounding portion of the platform 555. In this position the pressing zone 540 is enclosed other than an upper portion where the patient specific insert negative 800 is to be placed.

Figure 7(C) also shows a clocking element 578. The clocking element 578 provides pre-defined rotational position of the patient specific insert negative 800 relative to the housing 508. For some embodiments, specific orientation of the negative 800 relative to a bone press is useful for providing proper orientation or position of an augment on an asymmetrical bone graft. For example if a bone graft has an oblong, e.g., rectangular, cross section and the augment is to be provided at a specific region, e.g., along a long axis or a short axis of the bone graft, the position of the insert negative 800 relative to the housing 508 should be pre-defined to assure that the negative 800 produces the augment in the desired location. In other embodiments it is sufficient that the rotational position of the negative 800 relative to the housing 508 or the pressing zone 540 is sufficient to prevent circumferential compression which could result in shearing of the bone graft blank B. In one embodiment the housing has a cylindrical configuration with the inner periphery of the housing 508 being circular. At one or more locations of the inner periphery the periphery extends outwardly to form a longitudinal recess or channel 580 in the inner surface. The longitudinal channel 580 allows a projection 582 on the outer surface of the patient specific insert negative 800 to be received and to slide as the negative 800 is advanced in the housing 508. The channel 580 and the projection 582 engage to prevent relative rotation of the negative 800 and the housing 508 during such advancement. In another embodiment, the clocking element 578 can include channels on the patient specific insert negative 800 and projections on the housing 508. The channels or projections extends or extend from an upper surface of the housing 508 toward the lower surface thereof. The channel(s) or projection(s) can extend entirely between the upper and lower portions of the housing 508. Although the illustrated embodiment has only one channel 580, the clocking element 578 could include a plurality of channels 580 or projections if the purpose of the clocking element 578 is only for preventing rotation during movement of the patient specific insert negative 800.

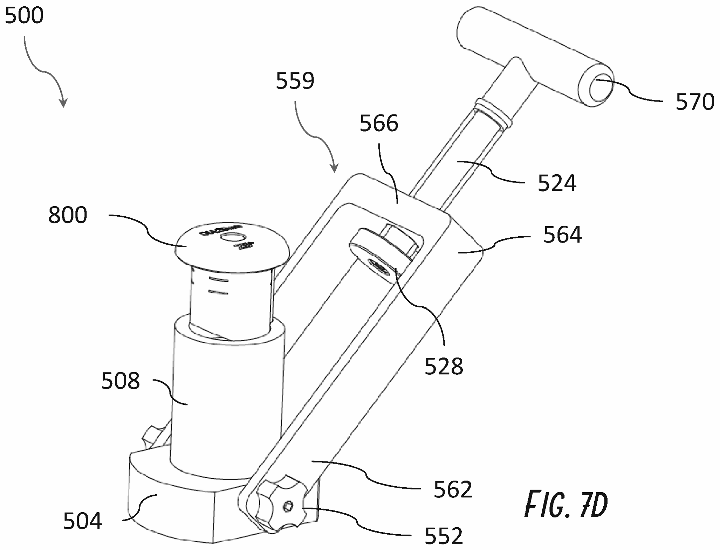

Figure 7(D) shows the insert negative 800 placed into the housing 508. The projection 582 is received in the channel 580. This provides a specific rotational position and also prevents or limits circumferential compression.

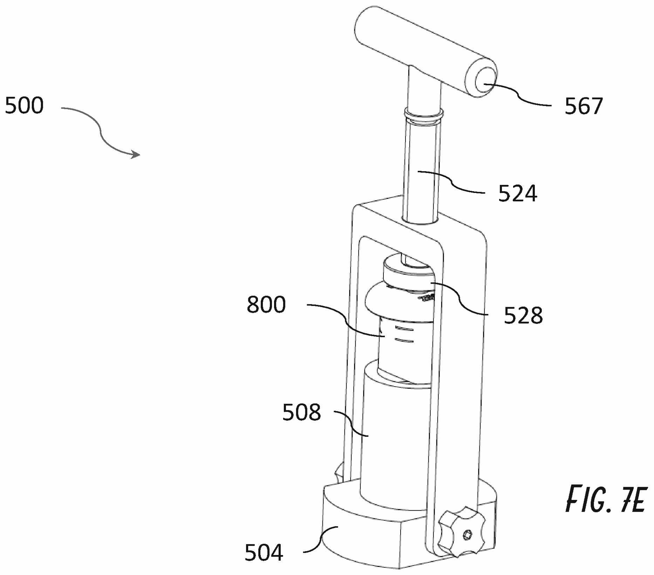

Figure 7(E) shows the compression plate 528 moved into contact with an upper surface 840 of the patient specific insert negative 800. After the compression plate 528 is in such contact, the handle 567 can be actuated to move the ram 524 and the compression plate 528 coupled therewith toward the base 504 to create compression of the bone graft blank B. Such compression can begin to compress the bone graft blank B. Further compression can cause the bone graft blank B to be reshaped. The reshaping of the bone graft blank can cause the medial surface of the bone graft blank B to form a shape matching the shape of the glenoid surface either as it is prior to surgery or after a prescribed amount and type of reaming. After the compression and/or reshaping the bone graft blank B is formed into a patient specific bone graft B'.

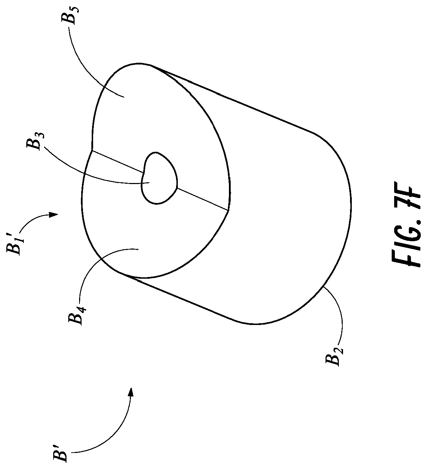

Figure 7(F) shows that in one example the patient specific bone graft B' has a first side that is or can be coupled with the prosthesis component 10' and a second side. The patient specific bone graft B' configured for the shoulder will have a contoured surface B₁' that corresponds to a shape of a glenoid. The surface B₁' is a patient specific bone facing portion, e.g., a portion that is configured to face glenoid of a patient. That is, the contoured surface B₁' is a negative of the surface of the glenoid. The contoured surface B₁' can have a first planar portion B₄ and a second planar portion B₅. The planar portions B₄, B₅ are configured to be nested in and to closely match the surfaces of the glenoid that are unaltered or that have been prepared by minimal reaming or other preparation method. The patient specific bone graft B' has a body that extends between the surface B₁' and a surface B₂ opposite the surface B₁'. The body of the bone graft B' is configured to adjust the spacing of a prosthesis component coupled with the side or surface B₂ from a surface of the bone to which the contoured surface B₁ or patient specific bone facing potion is coupled. The specific bone graft B' generally is compressed, as discussed herein within any of the bone presses herein. The specific bone graft B' can be formed from a single, monolithic piece of bone or from a plurality of pieces of bone. The specific bone graft B' can comprise natural bone matter or synthetic bone matter. The specific bone graft B' can include a lumen B₃ therethrough configured to receive a portion of a prostheses, such as the component 10' as shown herein.

Figure 9 shows another example of a bone press 600 that can be used to compress or reshape a bone graft blank B. The bone press 600 is similar to the bone press 500 except as described differently below. The bone press 600 includes a pressing zone 604 defined within a housing 608. The housing 608 includes a first shell 612 and a second shell 616. The first shell 612 comprises at least a portion of a base 620 configured to support either directly or indirectly the advancement of the patient specific insert negative 800. In the illustrated example, the first shell 612 includes a first portion of the base and the second shell 616 comprises a second portion of the base 620. In a variation the base 620 is disposed on only one of either the first shell 612 or the second shell 616.

The first shell 612 and the second shell 616 meet at an interface, which can be a planar interface. Figures 9 and 10 show that each of the first shell and the second shell 612, 616 comprises a portion of an access aperture 624 for receiving a shaft 692 of the impactor 690. The aperture 624 allows the impactor 690 to pass through the base 620 into the pressing zone 604 within the bone press 600.

The shells 612, 616 can each have a partially cylindrical configuration. For example a cylinder portion 628 can extend away from the portion of the base 620 formed by the shell 612 toward an end of the bone press 600 opposite the base. A cylinder portion 632 can extend away from the portion of the second shell 616 toward the opposite end. The cylinder portions 628, 632 can be half cylinders in one embodiment. The inside surfaces of the cylinder portions of the shells 612, 616 form the pressing zone 604. The outside surfaces of the cylinder portions are threaded to enable advancement of an actuator 636 as discussed below.

The shells 612, 616 can have one or more slots 640 formed therein. The slots 640 are configured to permit a mechanical connection between the actuator 636 and a compression plate 644 disposed in the pressing zone 604. In various examples there may be one, two, three, four, five, six, or more slots 640. In the illustrated example, there are four slots 640. Each of the shells 612, 616 has one slot 640 formed entirely therein. A slot 640 is formed on each side of the bone press along the interface between the shells 612, 616.

The compression plate 644 includes a central portion 652 that can be disposed inside the pressing zone 604 of the bone press 600 and force transfer projection 656 extending peripherally from the central portion 652. The compression plate has a force transfer projection 656 for at least one of the slots 640. The compression plate 644 can have one force transfer projection 656 for each of the slots 640, as illustrated in the Figure 11(C). By disposing the force transfer projections 656 through the slots 640 the force transfer projections can be engaged by the actuator 636 as discussed below. The projections 656 can comprise an enlarged lobe disposed outside the slot 640 and a narrow portion between the lobe and the central portion 652.

The actuator 636 comprises a cylindrical portion 664 at a first end 668 that includes a threaded interior 670. The first end has a distal face 671 that acts on the compression plate 644. The interior threads are configured to engage the exterior threads disposed along the shells 612, 616. The threads on the shells 612, 616 and on the interior 670 enable advancement of the actuator 636 along the shells. The advancement of the actuator 636 engage the compression plate 644 to push the compression plate into the bone graft blank B to compress and/or re-shape the bone graft blank. The actuator 636 has an enlarged hand grip 672. The hand grip 672 can include one or more enlarged members that the hand grip 672 can engage. The hand grip 672 can help increase the torque by extending farther than does the cylindrical portion 664 away from an axis of rotation of the actuator 636 about the shells 612, 616.

Figures 11(A)-11(E) illustrate the use of the bone press 600. The impactor 690 has a first end with a handle 678 and a second end with engagement features 680 to connect the impactor 690 with the component 10'. Figures 11(B) shows that the user can therefore connect the lateral side of the component 10' to the engagement features 680 of the impactor 690. Thereafter the bone graft blank B can be coupled with the component 10'. For example, thereafter lumen B₃ of the bone graft blank B can be placed over the anchor member 12'. The anchor member 12' can be inserted into the lumen B₃ from the lateral side B₂ of the bone graft blank B until the lateral side of the bone graft blank B engaged the medial side of the component 10'.

Figure 11(C) shows that after the component 10' and the bone graft blank B are coupled with the impactor 690 these components can be coupled with the bone press. For instance, the shaft 692 of the impactor 690 can be laid in a lower portion of the access aperture 624. When so placed, the component 10' and the bone graft blank B are disposed in the inside portion of the first shell 612. The lateral side of the portion of the impactor 690 carrying the engagement features 680 (see Figure 11(A)) can be placed in the first shell 612 such that the lateral side of the impactor is in direct contact with the base 620. Thereafter the patient specific insert negative 800 and the compression plate 644 are placed in the pressing zone 604, e.g., into the interior surface of the first shell 612. Specifically, the keyed projection 844 on the second side 806 of the negative can be received in a corresponding slot 657 of the compression plate 644. In one technique the keyed projection 844 is received in the slot 657 and thereafter the slot 657 and the plate 644 are placed in the first shell 612 such that the contoured surface 808 of the insert negative 800 faces the bone graft blank B and such that the bone graft blank B is between the base 620 and the compression plate 644. Figure 11(D) shows the foregoing arrangement.

Following placement as in Figure 11(D), a block 682 coupled with the second shell 616 is placed over a bottom portion of the base 620. The block 682 can be manipulated by a handle 684 coupled with the block 682. The block 682 can be coupled with a bottom portion of the base 620 in a suitable fashion. For example, a plurality of projections 686 disposed on the lower side of the block 682 can be received in a corresponding plurality of apertures 688 formed on the lower side of the base 620. The illustrated example shows that three projections 686 can be received in three corresponding apertures 688 shown in the illustrated example.

Figure 11(E) shows the second shell 616 coupled with the first shell 612. Thereafter the actuator 636 can be threaded onto the free end of the shells 612, 616. The threading of the actuator onto the shells 612, 616 is facilitated by the hand grip 672. Further threading, as indicated by arrow A (see Figure 10), causes the projections 656 of the compression plate 644 to be engaged by the distal face 671 of the actuator 636 opposite the hand grip 672. Further threading causes the actuator 636 to engage the projections 656 moving the compression plate 644 toward the bone graft blank B. After compression has begun further advancement of the actuator 636 can cause the bone graft blank B to be reshaped such that the bone graft blank can be re-formed from a generic shape to a shape specifically configured for a particular patient, as discussed above.

Figures 5-7E illustrate that the bone press 500 provides the benefit of high compression capability due to the threaded connection between the ram 534 with the benefit of compressing and/or reshaping the bone graft blank B into the patient specific bone graft right on the component 10'. By forming the bone graft on the component 10', the surgeon or another user can avoid transferring bone graft to the component 10' which would require an additional step and/or could result in damage to the bone graft.

Figure 9-11(E) illustrate that the bone press 600 provides the benefit of high compression capability due to the threaded connection between the actuator 636 and the shells 612, 616 with the benefit of having, pre-loaded onto the impactor 690, the component 10' and the formed patient specific bone graft formed from the bone graft blank B in the bone press 600. As a result, the surgeon is not required to load the bone graft and the component 10' onto the impactor 690. By forming the bone graft and the component 10' preloaded on the impactor the surgeon or another medical professional can avoid transferring the bone graft and the component 10' to the impactor 690 which would require an additional step and/or could result in the bone graft being displaced on or from the component 10'.

Figures 12-14(B) show another bone press 700 that can be used to form a patient specific bone graft from a bone graft blank B. The bone press 700 provides a number of advantages, including forming the bone graft on the impactor 690 and enabling formation of the bone graft through a single pre-defined motion among other advantages.

The bone press 700 includes a compression plate 702, a base 704, a housing 708, and an actuator 712. The compression plate 702 comprises a rigid plate-like configuration. The compression plate 702 has a first side 720 with a platform 722 and a second side 724 opposite the first side 720. The compression plate 702 has lateral sides 728 to which the actuator 712 is coupled. The actuator 712 is coupled to the compression plate 702 at the lateral sides 728 by a plurality of pins 730, but threaded members or other connection devices could be used. The first side 720 includes a keyed interface that can include a linear protrusion 732 that retains the patient specific insert negative 800 in a pre-defined rotational position relative to the bone graft blank B. The keyed interface also can include a peg 733 projecting away from the first side 720 of the compression plate 702 and away from the linear protrusion 732 toward the base 704. The patient specific insert negative 800 has a corresponding groove or slot 734. The slot 734 is sized to receive the protrusion 732 of the interface. If provided, the peg 733 is received in an aperture, recess or a lumen 850 of the patient specific insert negative 800 (See Figure 13). Other couplings could be provided between the patient specific insert negative 800 and the compression plate 702. For example, a plurality of pegs could be provided projecting from the first side 720 to mate in only one orientation with the patient specific insert negative 800. The pegs could be on the patient specific insert negative 800, to mate with apertures in the compression plate 702. The interface 732 could be on the patient specific insert negative 800 to mate with the groove or slot 734 formed on the compression plate 702. Other variations are also possible.

The base 704 has a first side 742 and a second side 744. The base 704 is disposed opposite the compression plate 702 in the bone press 700. The second side 744 faces the compression plate 702. The housing 708 extends between the base and the compression plate. The housing 708 has a first end 746 that contacts the compression plate 702 and a second end 748 that contact the base 704. A pressing zone 750 is formed between the patient specific insert negative 800 (mounted on the compression plate 702), the base 704, and the housing 708. As discussed herein, the pressing zone 750 is a place where compression of the bone graft blank B can occur. The base 704 also includes an access path 754 for receiving the impactor 690. The access path 754 preferably extends from one of the sides of the base 704, e.g., from a top side of the base 704. The access path can comprise a U-shaped recess formed form the top side of the base to a central portion of the base, e.g., to a position aligned with a central longitudinal axis of the pressing zone 750.

The actuator 712 comprises a pair of bars 762 disposed on opposite sides of the pressing zone 750. The bars 762 are pivotably coupled with the base 704. Pins 764 extend through the bars 762 and into the base. The pins 764 permit the bars to rotate from an upright position, shown in Figure 12, to inclined position, one of which is shown in Figure 13. The bars 760 are also rigidly connected by a transverse bar 768. The bars 760 are also connected by a force transfer rod 769. Movement between the pins 764 and the bars 762 is limited to rotation in some examples.

The force transfer rod 769 is able to pivot relative to the bars 760 and also can slide along slots 770 formed in the bars. The slots 770 are formed in each bar 760, are parallel to each other, and have the same length in some examples. Figures 12-14(B) all show the force transfer rod 769 at an end of the slot corresponding to a state of compression. The force transfer rod 769 can move toward or to the opposite end of the slot 770, which corresponds to a more open configuration of the bone press 700. In one embodiment, the slots 770 are eliminated and the bars 760 can be pivotably coupled with a force transfer member such without any movement of the force transfer member relative to the bars 760. The actuator 712 also includes a handle 772 that can be grasped by a user in order to move the actuator 712 between an upright position, as in Figure 12 and an inclined position as in Figure 13. The upright position is one in which compression can occur in the pressing zone 750. The inclined position is one in which compression is reduced or eliminated compared to the upright position. In some inclined position the pressing zone 750 can be opened and accessed to load the component 10' and/or the bone graft blank B.

Figures 14(A)-14(B) show how the bone press 700 can be used. In the illustrated example, the bone press 700 is configured to compress and reshape a bone graft blank B mounted on the impactor 690. Prior to compressing the bone graft blank B, the actuator 712 is placed in an inclined position, as shown in Figure 13-14B. Figure 13 shows that bone press 700 can receive the component 10' as well as the blank B. Accordingly, in one variation of the bone press 700 the component 10' and the bone graft blank B can be mounted to the impactor 690 as shown in Figures 11(A) - 11(B). The combination of the impactor 690, the component 10' and the bone graft blank B can be coupled with the base 704. In one embodiment, the shaft 692 is inserted into the access path 754. Thereafter, the component 10' can be mounted to engagement features 680 of the impactor 690 at a location between the base 704 and the compression plate 702. A bone graft blank B is then advanced over the anchor member 12'.

The pressing zone 750 then can be formed by placing the housing 708 over the bone graft blank B. The insert negative 800 can be mounted to the peg 733. The pressing zone 750 can be enclosed by placing the patient specific insert negative 800 into the housing 708 as shown in Figure 13.

After the pressing zone 750 is enclosed, the actuator can be moved from an inclined position, as in Figures 13-14(B), toward an upright position as shown in Figure 12. Figure 13 shows a gap between the patient specific insert negative 800 and the medial side B₁ of the bone graft blank B. A first movement of the actuator 712 from the inclined position of Figure 13 toward the upright position of Figure 12 the gap is closed and contact between the patient specific insert negative 800 and the medial side B₁ of the bone graft blank B. Further movement causes compression of the bone graft blank B. After compression of the bone graft blank B, further movement of the actuator 712 reshapes the bone graft blank B. The reshaping can be such that the bone graft blank B is formed into a patient specific bone graft.

The bone press 700 provides the advantage of transforming the bone graft blank B into a patient specific bone graft without requiring a repeating motion, such as threading one member relative to another. Rather, the transformation can result from a single motion from an inclined configuration (as in Figures 13-14(B) or more inclined) to an upright position as in Figure 12. The arrangement of the bars 760, 762 also can be arranged to create a great deal of compression in the pressing zone 750. The amount of compression can be tailored by the location of the force transfer member 769 and the handle 772.

### Terminology

Although certain embodiments have been described herein with respect to an anatomic component or a reverse component, the implants and methods described herein can interchangeably use any articular component, including the anatomic and reverse components described herein, as the context may dictate.

As used herein, the relative terms "proximal" and "distal" shall be defined from the perspective of the implant. Thus, proximal refers to the direction of the articular component and distal refers to the direction of the base plate when the implant is assembled.

Note that the terms "first" and "second'" articular components can be used interchangeably and to refer to the anatomic components or the reverse components. Accordingly, the "first" and "second" openings can be used interchangeably and to refer to any one of the openings in the baseplate.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments.

The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

The terms "approximately," "about," and "substantially" as used herein represent an amount close to the stated amount that still performs a desired function or achieves a desired result. For example, the terms "approximately," "about," and "substantially" may refer to an amount that is within less than 10% of the stated amount, as the context may dictate. As an example, in certain embodiments, the term "generally perpendicular" refers to a value, amount, or characteristic that departs from exactly perpendicular by less than about 10 degrees.

Although certain embodiments and examples have been described herein, it will be understood by those skilled in the art that many aspects of the glenoid implants shown and described in the present disclosure may be differently combined and/or modified to form still further embodiments or acceptable examples. All such modifications and variations are intended to be included herein within the scope of the invention providing they fall within the scope of the claims. A wide variety of designs and approaches are possible. No feature, structure, or step disclosed herein is essential or indispensable.

Some embodiments have been described in connection with the accompanying drawings. However, it should be understood that the figures are not drawn to scale. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

For purposes of this disclosure, certain aspects, advantages, and novel features are described herein. It is to be understood that not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Moreover, while illustrative embodiments have been described herein, the scope of any and all embodiments having equivalent elements, modifications, omissions, combinations (e.g., of aspects across various embodiments), adaptations and/or alterations as would be appreciated by those in the art based on the present disclosure. The limitations in the claims are to be interpreted broadly based on the language employed in the claims and not limited to the examples described in the present specification or during the prosecution of the application, which examples are to be construed as non-exclusive. Further, the actions of the disclosed processes and methods may be modified in any manner, including by reordering actions and/or inserting additional actions and/or deleting actions. It is intended, therefore, that the specification and examples be considered as illustrative only, with the scope of the invention being defined by the claims.

Any methods disclosed herein need not be performed in the order recited. The methods disclosed herein include certain actions taken by a practitioner; however, they can also include any third-party instruction of those actions, either expressly or by implication. For example, actions such as "inserting a base plate into a glenoid cavity" include "instructing insertion of a base plate into a glenoid cavity."

## Claims

1. A joint prosthesis spacer (2, B'), comprising:
a first side (2B, B2) to be facing away from a bone of a patient;
a second side (2C, B1') of the joint prosthesis spacer opposite the first side (B2), the second side (2C, B1') comprising a patient specific bone facing portion to face the bone of the patient; and
a bone graft body being a generally circular body extending between the first side (2B, B2) and the second side (2C, B1'), the bone graft body configured to be positioned between a prosthesis component (10, 10'), coupled with the first side, and a surface of the bone to which the patient specific bone facing portion is to be coupled to adjust the spacing between the prosthesis component and said surface of the bone thereby displacing an effective bone surface for coupling with the prosthesis component laterally outward to the first side;
wherein the bone graft body of the joint prosthesis spacer (2, B') is a single piece of natural bone that has been compressed axially from a first state to an irreversibly deformed second state.

2. The joint prosthesis spacer of Claim 1, wherein a lumen (B3) extends through the bone graft body from the first side (2B, B2) to the second side (2C, B1').

3. The joint prosthesis spacer of any one of Claims 1 to 2, wherein the body has a non-constant thickness.

4. The joint prosthesis spacer of Claim 3, wherein a thickness (L1) at an inferior portion of the spacer (2, B') is smaller than a thickness (L2) at a superior portion of the spacer.

5. The joint prosthesis spacer of any one of Claims 1 to 3, wherein the second side (B1') comprises a first planar portion (B4) and a second planar portion (B5).

6. A system, comprising:
The joint prosthesis spacer (2, B') of any one of the preceding claims; and
a prosthesis component (10, 10').

7. The system of Claim 6, wherein the prosthesis component (10, 10') comprises an anchor member (12, 12'), the spacer (2, B') including a lumen (B3) that extends through the bone graft body from the first side (2B, B2) to the second side (2C, B1'), the lumen sized and configured to receive the anchor member (12, 12') such that the spacer is configured to be advanced over the anchor member (12, 12').

8. The system of Claim 7, wherein the prosthesis component (10, 10') comprises a concave surface (32).

9. The system of Claim 8, wherein the prosthesis component (10, 10') comprises a convex articular surface (11A).

10. A system, comprising:
the joint prosthesis spacer (2, B') of any one of the Claims 1 to 5; and
a patient specific insert negative (800) comprising a contoured surface (808) configured to correspond to a patient specific joint surface.

11. The system of claim 7, wherein the anchor member (12) includes a free end that is configured to be anchored in a scapula (S) when the prosthesis component (10, 10') is implanted.

12. The system of any of claims 7 or 11, wherein the first side (2B, B2) of the joint prosthesis spacer is covered by the prosthesis component (10, 10') when the prosthesis component (10, 10') and the joint prosthesis spacer are implanted.

13. The system of any of claims 7, 11, or 12, further comprising:
a patient specific insert negative (800) comprising a contoured surface (808), the patient specific insert negative configured to be mounted on or in a bone press.

## Patentansprüche

1. Ein Gelenkprothesenspacer (2, B'), umfassend:
eine erste Seite (2B, B2), die von einem Knochen eines Patienten weggewandt sein soll;
eine zweite Seite (2C, B1') des Gelenkprothesenspacers gegenüber der ersten Seite (B2), wobei die zweite Seite (2C, B1') einen patientenspezifischen knochenzugewandten Abschnitt umfasst, der dem Knochen des Patienten zugewandt sein soll; und
einen Knochentransplantatkörper, der ein im Allgemeinen kreisförmiger Körper ist, der sich zwischen der ersten Seite (2B, B2) und der zweiten Seite (2C, B1') erstreckt, wobei der Knochentransplantatkörper dazu eingerichtet ist, zwischen einer mit der ersten Seite gekoppelten Prothesenkomponente (10, 10') und einer Oberfläche des Knochens, mit der der patientenspezifische knochenzugewandte Abschnitt gekoppelt werden soll, positioniert zu werden, um den Abstand zwischen der Prothesenkomponente und der besagten Oberfläche des Knochens anzupassen und dadurch eine effektive Knochenoberfläche zur Kopplung mit der Prothesenkomponente lateral nach außen zur ersten Seite zu verlagern;
wobei der Knochentransplantatkörper des Gelenkprothesenspacers (2, B') ein einzelnes Stück natürlichen Knochens ist, das axial von einem ersten Zustand in einen irreversibel verformten zweiten Zustand komprimiert worden ist.

2. Der Gelenkprothesenspacer nach Anspruch 1, wobei sich ein Lumen (B3) durch den Knochentransplantatkörper von der ersten Seite (2B, B2) zur zweiten Seite (2C, B1') erstreckt.

3. Der Gelenkprothesenspacer nach einem der Ansprüche 1 bis 2, wobei der Körper eine nicht konstante Dicke aufweist.

4. Der Gelenkprothesenspacer nach Anspruch 3, wobei eine Dicke (L1) an einem unteren Abschnitt des Spacers (2, B') kleiner ist als eine Dicke (L2) an einem oberen Abschnitt des Spacers.

5. Der Gelenkprothesenspacer nach einem der Ansprüche 1 bis 3, wobei die zweite Seite (B1') einen ersten ebenen Abschnitt (B4) und einen zweiten ebenen Abschnitt (B5) umfasst.

6. Ein System, umfassend:
Den Gelenkprothesenspacer (2, B') nach einem der vorhergehenden Ansprüche; und eine Prothesenkomponente (10, 10').

7. Das System nach Anspruch 6, wobei die Prothesenkomponente (10, 10') ein Ankerelement (12, 12') umfasst, wobei der Spacer (2, B') ein Lumen (B3) aufweist, das sich durch den Knochentransplantatkörper von der ersten Seite (2B, B2) zur zweiten Seite (2C, B1') erstreckt, wobei das Lumen so bemessen und dazu eingerichtet ist, das Ankerelement (12, 12') aufzunehmen, sodass der Spacer dazu eingerichtet ist, über das Ankerelement (12, 12') vorgeschoben zu werden.

8. Das System nach Anspruch 7, wobei die Prothesenkomponente (10, 10') eine konkave Oberfläche (32) umfasst.

9. Das System nach Anspruch 8, wobei die Prothesenkomponente (10, 10') eine konvexe Gelenkfläche (11A) umfasst.

10. Ein System, umfassend:
den Gelenkprothesenspacer (2, B') nach einem der Ansprüche 1 bis 5; und
ein patientenspezifisches Einsatznegativ (800), umfassend eine konturierte Oberfläche (808), die dazu eingerichtet ist, einer patientenspezifischen Gelenkfläche zu entsprechen.

11. Das System nach Anspruch 7, wobei das Ankerelement (12) ein freies Ende aufweist, das dazu eingerichtet ist, in einem Schulterblatt (S) verankert zu werden, wenn die Prothesenkomponente (10, 10') implantiert ist.

12. Das System nach einem der Ansprüche 7 oder 11, wobei die erste Seite (2B, B2) des Gelenkprothesenspacers von der Prothesenkomponente (10, 10') bedeckt ist, wenn die Prothesenkomponente (10, 10') und der Gelenkprothesenspacer implantiert sind.

13. Das System nach einem der Ansprüche 7, 11 oder 12, ferner umfassend:
ein patientenspezifisches Einsatznegativ (800), umfassend eine konturierte Oberfläche (808), wobei das patientenspezifische Einsatznegativ dazu eingerichtet ist, an oder in einer Knochenpresse montiert zu werden.

## Revendications

1. Un espaceur de prothèse articulaire (2, B'), comprenant :
un premier côté (2B, B2) destiné à être orienté à l'opposé d'un os d'un patient ;
un second côté (2C, B1') de l'espaceur de prothèse articulaire opposé au premier côté (B2), le second côté (2C, B1') comprenant une portion spécifique au patient faisant face à l'os, destinée à faire face à l'os du patient ; et
un corps de greffon osseux étant un corps généralement circulaire s'étendant entre le premier côté (2B, B2) et le second côté (2C, B1'), le corps de greffon osseux étant configuré pour être positionné entre un composant de prothèse (10, 10'), couplé au premier côté, et une surface de l'os à laquelle la portion spécifique au patient faisant face à l'os doit être couplée pour régler l'espacement entre le composant de prothèse et ladite surface de l'os, déplaçant ainsi une surface osseuse effective destinée à être couplée au composant de prothèse latéralement vers l'extérieur jusqu'au premier côté ;
dans lequel le corps de greffon osseux de l'espaceur de prothèse articulaire (2, B') est un seul morceau d'os naturel qui a été comprimé axialement d'un premier état à un second état déformé de manière irréversible.

2. L'espaceur de prothèse articulaire selon la revendication 1, dans lequel une lumière (B3) traverse le corps de greffon osseux du premier côté (2B, B2) au second côté (2C, B1').

3. L'espaceur de prothèse articulaire selon l'une quelconque des revendications 1 à 2, dans lequel le corps présente une épaisseur non constante.

4. L'espaceur de prothèse articulaire selon la revendication 3, dans lequel une épaisseur (L1) au niveau d'une portion inférieure de l'espaceur (2, B') est inférieure à une épaisseur (L2) au niveau d'une portion supérieure de l'espaceur.

5. L'espaceur de prothèse articulaire selon l'une quelconque des revendications 1 à 3, dans lequel le second côté (B1') comprend une première portion plane (B4) et une seconde portion plane (B5).

6. Un système, comprenant :
L'espaceur de prothèse articulaire (2, B') selon l'une quelconque des revendications précédentes ; et un composant de prothèse (10, 10').

7. Le système selon la revendication 6, dans lequel le composant de prothèse (10, 10') comprend un élément d'ancrage (12, 12'), l'espaceur (2, B') comprenant une lumière (B3) qui traverse le corps de greffon osseux du premier côté (2B, B2) au second côté (2C, B1'), la lumière étant dimensionnée et configurée pour recevoir l'élément d'ancrage (12, 12') de sorte que l'espaceur soit configuré pour être avancé sur l'élément d'ancrage (12, 12').

8. Le système selon la revendication 7, dans lequel le composant de prothèse (10, 10') comprend une surface concave (32).

9. Le système selon la revendication 8, dans lequel le composant de prothèse (10, 10') comprend une surface articulaire convexe (11A).

10. Un système, comprenant :
l'espaceur de prothèse articulaire (2, B') selon l'une quelconque des revendications 1 à 5 ; et
un négatif d'insert spécifique au patient (800) comprenant une surface profilée (808) configurée pour correspondre à une surface articulaire spécifique au patient.

11. Le système selon la revendication 7, dans lequel l'élément d'ancrage (12) comprend une extrémité libre qui est configurée pour être ancrée dans une scapula (S) lorsque le composant de prothèse (10, 10') est implanté.

12. Le système selon l'une quelconque des revendications 7 ou 11, dans lequel le premier côté (2B, B2) de l'espaceur de prothèse articulaire est recouvert par le composant de prothèse (10, 10') lorsque le composant de prothèse (10, 10') et l'espaceur de prothèse articulaire sont implantés.

13. Le système selon l'une quelconque des revendications 7, 11, ou 12, comprenant en outre :
un négatif d'insert spécifique au patient (800) comprenant une surface profilée (808), le négatif d'insert spécifique au patient étant configuré pour être monté sur ou dans une presse à os.
